(19) Europäisches Patentamt
European Patent Office
Office européen des brevets

(11) Veröffentlichungsnummer: **0 158 312**
**B1**

(12) # EUROPÄISCHE PATENTSCHRIFT

(45) Veröffentlichungstag der Patentschrift: **12.09.90**

(21) Anmeldenummer: **85104247.3**

(22) Anmeldetag: **11.04.85**

(51) Int. Cl.$^5$: **C 07 D 217/14,** A 61 K 31/47, C 07 D 217/04

(54) **1-Cyclohexylr-3,4-di-hydror-isochinolinderivate, Verfahren zu ihrer Herstellung und diese enthaltende Arzneimittel.**

(30) Priorität: **11.04.84 HU 139384**

(43) Veröffentlichungstag der Anmeldung: **16.10.85 Patentblatt 85/42**

(45) Bekanntmachung des Hinweises auf die Patenterteilung: **12.09.90 Patentblatt 90/37**

(84) Benannte Vertragsstaaten: **AT BE CH DE FR GB IT LI NL SE**

(56) Entgegenhaltungen: **GB-A- 488 423**

**JOURNAL OF MEDICINAL CHEMISTRY, Band 22, Nr. 4, 1979, Seite 348, American Chemical Society, Washington, D.C., US; V. St. GEORGIEV et al.: "Drug-induced modifications of the immune response. I. Substituted 1-phenylisoquinolines"**

(73) Patentinhaber: **RICHTER GEDEON VEGYESZETI GYAR R.T.**
**Gyömröi ut 19-21**
**H-1475 Budapest X (HU)**

(72) Erfinder: **Bernáth, Gábor, Dr. Dipl.-Chem.**
**Mérei u. 8**
**H-6722 Szeged (HU)**
Erfinder: **Kóbor, Jenö, Dr.**
**Jósika u. 7**
**H-6722 Szeged (HU)**
Erfinder: **Lázár, János, Dr. Dipl.-Pharm.**
**Szamos u. 18/A**
**H-6723 Szeged (HU)**
Erfinder: **Motika, Gábor, Dr. Dipl.-Chem.**
**Vajda u. 18/A**
**H-6723 Szeged (HU)**
Erfinder: **Ezer, Elemér, Dipl.-Chem.**
**Lupény u. 6-8**
**H-1026 Budapest (HU)**
Erfinder: **Hajós, György, Dr. Dipl.-Pharm.**
**Gábor Aron u. 59**
**H-1026 Budapest (HU)**
Erfinder: **Pálosi, Eva, Dr.**
**Vend u. 21**
**H-1025 Budapest (HU)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(72) Erfinder: **Dénes, László, Dr. Dipl.-Pharm.**
**Szél u. 19**
**H-1035 Budapest (HU)**
Erfinder: **Szporny, László, Dr.**
**Szabolcska M. u. 7**
**H-1114 Budapest (HU)**

(74) Vertreter: **Beszédes, Stephan G., Dr.**
**Patentanwalt**
**Münchener Strasse 80a Postfach 1168**
**D-8060 Dachau (DE)**

**Beschreibung**

Die Erfindung betrifft neue 3,4-Di-[hydro]-isochinolin-derivate einschließlich ihrer Säureadditionssalze, ein Verfahren zu ihrer Herstellung und diese Verbindungen enthaltende Arzneimittel, insbesondere mit krampflösenden, schmerzstillenden, sedativ-hypnotischen, die Magensäuresekretion hemmenden und die Dauer des Alkoholschlafes verkürzenden Wirkungen.

Aus dem Schrifttum sind zahlreiche pharmazeutisch wirksame Verbindungen mit einem Isochinolingerüst als Grundstruktur bekannt (zum Beispiel Ehrhart-Ruschig, Arzneimittel; S. Ebel, Synthetische Arzneimittel, Verlag Chemie). Verbindungen mit Isochinolinstruktur, die in der 1-Stellung durch einem Cyclohexylring substituiert sind und antiallergische sowie ZNS-Eigenschaften besitzen, sind aus der GB—A—488423 beziehungsweise aus Journal of Med. Chem., Band 22, Nr. 4, 1979, Seite 348 bekannt. Von anderen Wirkungen ist in diesen Druckschriften jedoch keine Rede.

Der Erfindung liegt die Aufgabe zugrunde, neue 3,4-Di-[hydro]-isochinolinderivate mit überlegenen pharmakologischen, insbesondere krampflösenden, schmerzstillenden, sedativ-hypnotischen, die Magensäuresekretion hemmenden und die Dauer des Alkoholschlafes verkürzenden, Wirkungen, ein Verfahren zu ihrer Herstellung und diese enthaltende Arzneimittel zu schaffen.

Das Obige wurde überraschenderweise durch die Erfindung erreicht.

Gegenstand der Erfindung sind 1-[Cyclohexyl]-3,4-di-[hydro]-isochinolinderivate der allgemeinen Formel

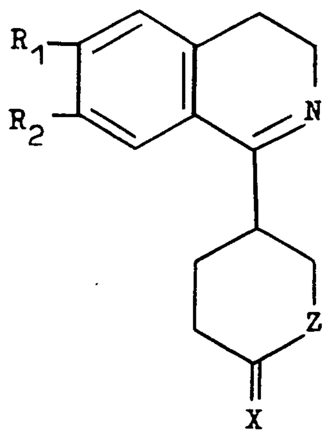

I,

worin

$R_1$ und $R_2$ unabhängig voneinander für Wasserstoff, Hydroxygruppen bezeihungsweise Alkoxyreste mit 1 bis 6 Kohlenstoffatom(en) stehen und entweder

X Sauerstoff bedeutet und

Z einen Rest der Formel

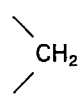

II,

oder einen Rest der allgemeinen Formel

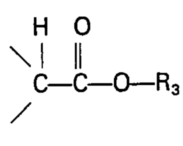

III,

in welchletzterer

$R_3$ für Wasserstoff oder einen Alkylrest mit 1 bis 6 Kohlenstoffatom(en) steht, darstellt oder

X einen Rest der allgemeinen Formel

$$=N—R_4$$ IV,

in welchletzterer $R_4$ für Wasserstoff oder eine Hydroxygruppe steht, bedeutet und

Z einen Rest der Formel

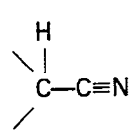

V,

einen Rest der Formel

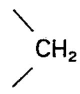

II,

oder einen Rest der allgemeinen Formel

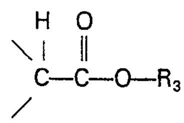

III,

in welchletzterere

$R_3$ für Wasserstoff oder einen Alkylrest mit 1 bis 6 Kohlenstoffatom(en) steht, darstellt, sowie ihre Säureadditionssalze.

Die Alkylreste, für die $R_3$ stehen kann, beziehungsweise welche in den Alkoxyresten, für die $R_1$ und/oder $R_2$ stehen können, vorliegen können, sind gesättigt und können geradkettig oder verzweigt sein. Beispiele sind Methyl-, Äthyl-, n-Propyl-, Isopropyl-, n-Butyl-, sek.-Butyl-, tert.-Butyl-, n-Pentyl-, Isopentyl-, n-Hexyl- und Isohexylreste.

Vorzugsweise sind die Alkoxyreste, für welche $R_1$ und/oder $R_2$ stehen können, solche mit 1 bis 4, insbesondere 1 oder 2, ganz besonders 1, Kohlenstoffatom(en). Dabei sind ganz besonders bevorzugt beide von $R_1$ und $R_2$ gleich.

Es ist auch bevorzugt, daß der Alkylrest, für welchen $R_3$ stehen kann, ein solcher mit 1 bis 4, insbesondere 1 oder 2, Kohlenstoffatom(en) ist.

Ganz besonders bevorzugte erfindungsgemäße Verbindungen sind 1 - [4' - (Oxo) - cyclohexyl] - 3,4 - di - [hydro] - 6,7 - di - [methoxy] - isochinolin, 1 - [{3' - (Methoxycarbonyl) - 4' - (oxo)} - cyclohex - 1' - yl] - 3,4 - di - [hydro] - 6,7 - di - [methoxy] - isochinolin, 1-[{3' - (Äthoxycarbonyl) - 4' - (oxo)} - cyclohex - 1' - yl] - 3,4 - di - [hydro] - 6,7 - di - [methoxy] - isochinolin, 1 - [{3' - Cyano) - 4' - (imino)} - cyclohex - 1' - yl] - 3,4 - di - [hydro] - 6,7 - di - [methoxy] - isochinolin, 1 - [Cyclohex - 1' - yl - 4' - (oxim)] - 3,4 - di - [hydro] - 6,7 - di - [methoxy] - isochinolin, und 1 - [{3' - (Methoxycarbonyl) - 4' - (imino)} - cyclohex - 1' - yl] - 3,4 - di - [hydro] - 6,7 - di - [methoxy] - isochinolin sowie ihre Hydrochloride.

Gegenstand der Erfindung ist auch ein Verfahren zur Herstellung der erfindungsgemäßen Verbindungen, welches dadurch gekennzeichnet ist, in an sich bekannter Weise

a) zur Herstellung von 1-[Cyclohexyl)-3,4-di-[hydro]-isochinolinderivaten der allgemeinen Formel I, bei welchen X für Sauerstoff steht und Z einen Rest der allgemeinen Formel III, in welcher $R_3$ einen Alkylrest mit 1 bis 6 Kohlenstoffatom(en) darstellt, bedeutet sowie $R_1$ und $R_2$ dieselben Bedeutungen wie oben haben, 4-[3',4'-Di-(hydro)-(2'H)-isochinolin-1'-yl]-heptan-1,7-di-[carbonsäure]-derivate der allgemeinen Formel

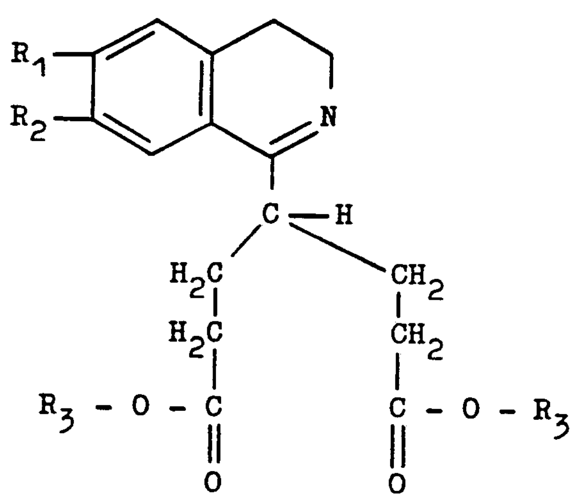

VI,

Es folgen Seiten 7 bis 28 der ursprünglichen Unterlagen vom 11. April 1985 worin $R_1$, $R_2$ und $R_3$ dieselben Bedeutungen wie oben haben, in Gegenwart von Alkalimetallen, Alkalimetallalkoholaten oder Alkalimetallamiden cyclisiert werden oder

b) zur Herstellung von 1-[Cyclohexyl]-3,4-di-[hydro]-isochinolinderivaten der allgemeinen Formel I, bei welchen X für einen Rest der allgemeinen Formel IV, in welcher $R_4$ Wasserstoff bedeutet, steht und Z einen Rest der Formel V darstellt, sowie $R_1$ und $R_2$ dieselben Bedeutungen wie oben haben, 4-[3',4'-Di-(hydro)-(2'H)-isochinolin-1'-yl]-heptan-1,7-di-[carbonsäurenitril]-derivate der allgemeinen Formel

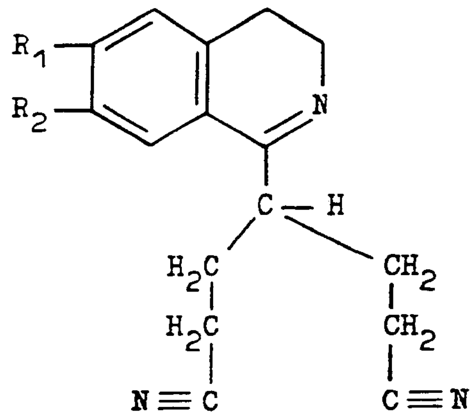

VII,

worin $R_1$ und $R_2$ dieselben Bedeutungen wie oben haben, in Gegenwart von Alkalimetallen, Alkalimetallalkoholaten oder Alkalimetallamiden cyclisiert werden,

worauf

gegebenenfalls nach der Variante a) erhaltene 1-[Cyclohexyl]-3,4-di-[hydro]-isochinolinderivate der allgemeinen Formel I, bei welchen Z, X, $R_1$, $R_2$ und $R_3$ die bei der Variante b) angegebenen Bedeutugen haben, zu den entsprechenden 1-[Cyclohexyl]-3,4-di-[hydro]-isochinolinderivaten der allgemienen Formel I, bei welchen Z für einen Rest der allgemeinen Formel III, in welcher $R_3$ Wasserstoff bedeutet, steht oder einen Rest der Formel II darstellt, hydrolysiert und/oder decarboxyliert werden und/oder

gegebenenfalls gegebenenfalls erhaltene 1-[Cyclohexyl]-3,4-di-[hydro]-isochinolinderivate der allgemeinen Formel I, bei welchen X für Sauerstoff steht und Z, $R_1$, $R_2$ und $R_3$ dieselben Bedeutungen wie oben haben, durch Umsetzen mit Hydroxylamin beziehungsweise Ammoniak zu den entsprechenden 1-[Cyclohexyl]-3,4-di-[hydro]-isochinolinderivaten der allgemeinen Formel I, bei welchen X einen Rest der allgemeinen Formel IV bedeutet, umgesetzt werden und/oder

gegebenenfalls in 1-[Cyclohexyl]-3,4-di-[hydro]-isochinolinderivaten der allgemeinen Formel I der beziehungsweise die Substituenten, für welche[n] $R_1$ und/oder $R_2$ steht beziehungsweise stehen, zu [einem] anderen Substituenten, welche[n] $R_1$ und/oder $R_2$ bedeuten kann, umgesetzt werden und/oder

gegebenenfalls die erhaltenen 1-[Cyclohexyl]-3,4-di-[hydro]-isochinolinderivate der allgemeinen Formel I in Säureadditionssalze überführt werden beziehungsweise gegebenenfalls die erhaltenen Säureadditionsalze der 1-[Cyclohexyl]-3,4-di-[hydro]-isochinolinderivate der allgemeinen Formel I in die freien 1-[Cyclohexyl]-3,4-di-[hydro]-isochinolinderivative der allgemeinen Formel I oder in andere Säureadditionssalze überführt werden.

Diejenigen der als Ausgangsstoffe benötigten Verbindungen VI und VII, in denen $R_1$ und $R_2$ für Wasserstoff stehen, sind bekannt (S. G. Agalyan, L. A. Nersesyun, Zs. A. Hanamiryan: Arm. Chim. Zsurn. *20*, 45 [1967]), und zum Beispiel aus den entsprechenden 1-Methyl-isochinolinderivaten herstellbar. Die als $R_1$ und $R_2$ andere Gruppen enthaltenden Verbindungen der allgemeinen Formeln VI und VII können auf literaturbekannte Weise hergestellt werden.

Ausgehend von Verbindungen der allgemeinen Formel VI können die als X ein Sauerstoffatom, als Z eine Gruppe der allgemeinen Formel $CH—COOR_3$ mit $R_3$ = Alkylgruppe mit 1—6 Kohlenstoffatomen enthaltenden Verbindungen der allgemeinen Formel I [Variante a)] unter den Bedingungen der Claisen-Kondensation hergestellt werden. Dazu werden die Diester der allgemeinen Formel VI in Gegenwart eines Alkalimetalls, Alkalimetallalkoholates oder Alkalimetallamids unter Abspaltung von Alkohol zu den entsprechenden Verbindungen der allgemeinen Formel I cyclisiert. Zur Esterkondensation wird vorzugsweise Natriummethylat oder -äthylat eingesetzt, jedoch verläuft die Reaktion auch in Gegenwart von metallischem Natrium oder Natriumamid. Die Umsetzung wird in Gegenwart eines hinsichtlich der Reaktanten inerten, vorzugsweise aromatischen organischen Lösungsmittels, zum Beispiel Benzol oder Toluol, bei erhöhter Temperatur, vorzugsweise am Siedepunkt des Reaktionsgemisches vorgenommen.

Die Variante b) ist, was die Reaktionsbedingungen anbetrifft, der Variante a) völlig analog. d.h. man verwendet auch hier ein Alkalimetall, dessen Alkoholat oder Amid, vorzugsweise Natrium, Natriumäthylat oder -methylat oder Natriumamid, insbesondere jedoch Natriummethylat oder -äthylat. Als Lösungsmittel wird auch hier ein hinsichtlich der Reaktanten inertes, vorzugsweise organisches aromatisches.

Lösungsmittel, zum Beispiel Benzol oder Toluol, gewählt, und die Reaktion wird bei erhöhter Temperatur, zweckmäßig bei Rückflußtemperatur, vorgenommen.

Die gemäß Variante a) erhaltenen Ketoester ($R_3$ = Alkylgruppe mit 1—6 Kohlenstoffatomen) können durch Hydrolyse, vorzugsweise unter alkalischen Bedingungen, zu den entsprechenden Ketocarbonsäuren umgesetzt werden. Die Ketoester beziehungsweise die entsprechenden Ketosäuren können unter den üblichen Bedingungen, durch Erwärmem decarboxyliert werden. Auf diese Weise werden diejenigen Verbindungen der allgemeinen Formel (I) erhalten, in denen Z für einen Rest der Formel II steht.

Verbindungen der allgemeinen Formel I, in denen X Sauerstoffatom bedeutet, können durch allgemein bekannte Oxoreagenzien zu den entsprechenden, an Stelle von X eine andere Gruppe enthaltenden Verbindungen umgesetzt werden. Zum Beispiel sind aus den Oxoverbindungen durch Umsetzen mit Hydroxylamin die entsprechenden Oximverbindungen, durch Umsetzen mit Ammoniak die entsprechenden Iminverbindungen erhältlich.

In den Verbindungen der allgemeinen Formel I, können die Substituenten $R_1$ und/oder $R_2$ gewünschtenfalls zu anderen, unter die bereits weiter oben gegebene Definition fallenden Substituenten umgesetzt werden. Aus den als $R_1$ und/oder $R_2$ eine Alkoxygruppe mit 1—6 Kohlenstoffatomen enthaltenden Verbindungen der allgemeinen Formel I können durch Desalkylierung die entsprechenden, als $R_1$ und/oder $R_2$ eine Hydroxylgruppe enthaltenden Verbindungen hergestellt werden. Die Desalkylierung wird in an sich bekannter Weise zum Beispiel durch Erwärmen mit Brom- oder Jodwasserstoff oder durch Zusatz von wasserfreiem Aluminiumchlorid vorgenommen. Umgekehrt können auch aus den als $R_1$ und/oder $R_2$ eine Hydroxylgruppe enthaltenden Verbindungen der allgemeinen Formel I die entsprechenden Alkoxyderivate hergestellt werden. Methylrest wird bevorzugt mit Diazomethan oder Dimethylsulfat, zum Einbringen höherer Alkylgruppen ist die Williamson-Synthese unter Verwendung von Alkyljodiden geeignet.

Aus den Verbindungen der allgemeinen Formel I können durch Umsetzen mit geeigneten Säuren Säureadditionssalze gebildet werden. Die Salzbildung wird in einem organischen Lösungsmittel, zum Beispiel einem aliphatischen Alkohol mit 1—6 Kohlenstoffatomen vorgenommen, indem man die Verbindung der allgemeinen Formel I auflöst und zu der Lösung so lange die entsprechende Säure oder deren mit einem Lösungsmittel bereitete Lösung gibt, bis das Gemisch schwach sauer reagiert. Das ausgefallene Säureadditionssalz wird in geeigneter Weise, zum Beispiel durch Filtrieren, abgetrennt.

Die erfindungsgemäßen Verbindungen der allgemeinen Formel I und ihre Säureadditionssalze können gewünschtenfalls einer weiteren Reinigung, zum Beispiel durch Umkristallisieren, unterzogen werden. Das zum Umkristallisieren eingesetzte Lösungsmittel wird in Abhängigkeit von der Löslichkeit und den Kristallisationseigenschaften des zu kristallisierenden Stoffes gewählt.

Ferner sind erfindungsgemäß Arzneimittel, welche 1 oder mehr erfindungsgemäße Verbindung(en) als Wirkstoff(e), zweckmäßigerweise zusammen mit 1 oder mehr üblichen pharmazeutischen Träger-, Streck- und/oder Hilfstoff(en), enthalten, vorgesehen. Die erfindungsgemäßen 1-[Cyclohexyl]-3,4-di-[hydro]-isochinolinderivate haben nämlich wertvolle pharmakologische, insbesondere krampflösende, schmerzstillende, sedativ-hypnotische, die Magensäuresekretion, emmende und die Dauer des Alkoholschalfes verkürzende, Wirkungen.

Die Wirkstoffe der allegmeinen Formel I und ihre physiologisch verträglichen Säureadditionssalze können mit den in der Arzneimittelherstellung üblichen, zur parenteralen oder enteralen Applikation geeigneten, nichttoxischen festen und/oder flüssigen Trägerstoffen und/oder Hilfsstoffen vermischt und zu Arzneimittelpräparaten formuliert werden. Als Trägerstoffe finden zum Beispiel Wasser, Gelatine, Lactose, Stärke, Pektin, Magnesiumstearat, Stearinsäure, Talk, Pflanzenöle (wie Erdnußöl und/oder Olivernöl) Verwendung. Die Wirkstoffe werden zu der üblichen Darreichungsformen formuliert, zum Beispiel festen Präparaten (abgerundeten oder eckigen Tabletten, Dragees, Kapseln, z.B. Hartgelatinekapseln, Pillen, Zäpfchen) oder flüssigen Präparaten (mit Öl oder Wasser bereiteten Lösungen, Suspensionen, Emulsionen, Sirupen, Weichgelatinekapseln, mit Öl oder wasser bereiteten injizierbaren Lösungen oder Suspensionen). Die Menge des festen Trägerstoffes kann in einem weiten Bereich variieren, eine Darreichungseinheit enthält vorzugsweise etwa 25 mg bis 1 g Trägerstoff. Die Präparate können gegebenenfalls die üblichen Hilfsstoffe, zum Beispiel Konservierungs-, Stabilisierungs-, Netz- und Emulgiermittel, Salze zum Einstellen des osmotischen Druckes, Puffer, Geruchs- und Geschmacksstoffe, enthalten. Die Präparate können weitere pharmazeutisch wertvolle, bekannte Verbindungen enthalten, ohne daß dadurch eine synergistische Wirkung einträte. Die Präparate werden vorzugsweise in Darreichungseinheiten formuliert, die der gewünschten Applikationsform entsprechen. Die Arzneimittelpräparate werden mittels der dafür üblichen Verfahren hergestellt, zum Beispiel durch Sieben, Mischen, Granulieren und Pressen der Komponenten oder durch Auflösen. Die Präparate können ferner weiteren, in der Arzneimittelindustrie üblichen Arbeitsgängen unterzogen, zum Beispiel sterilisiert werden.

Für die im folgenden geschliderten pharmakologischen Tests wurden CFLP-Mäuse (LATI) beiderlei Geschlechts und mit einem Gewicht von 18—22 g, ferner Han. Wistar-Ratten mit einem Gewicht von 160—180 g verwendet. Die zu testenden Substanzen wurden peroral in Dosen von 30 mg/kg in Form einer 5% Tween®80 enthaltenden Suspension eine Stunde vor Versuchsbeginn verabreicht.

## Testmethoden

1. Metrazolkrampf (MET) (an Mäusen)

Nach der Vorbehandlung wurden den Tieren 125 mg/kg Pentylentetrazol subkutan appliziert. Als geschützt wurden diejenigen Tiere betrachtet, die überlebten und bei denen der tonische Extensorkrampfanfall unterblieb. Die Beobachtungsdauer betrug eine Stunde (Everett, L. M. und Richards, R. K.: J. Pharmacol. Exp. Ther. *81*, 402, 1944).

2. Schmerzstillende Wirkung (FCS) (an Mäusen)

Eine Stunde nach der Vorbehandlung wurde den Tieren als Schmerzreiz 0,6 %ige Essigsäure intraperitoneal injiziert (0,3 ml). Die Haüfigkeit des Writhing-Syndroms wurde 30 Minuten lang registriert. Die durch die Behandlung auftretende Veränderung wurde zu den Durchschnittswerten der Kontrolle in Beziehung gesetzt und der Unterschied in Prozent ausgedrückt (Koster, R. et al., J. Pharmacol. Exp. Ther. *72*, 74 [1941]).

3. Akute Alkoholvergiftung (ETA)

Eine Stunde nach der Vorbehandlung wurden die Tiere mit Äthylalkohol in einer Dosis von 3,5 g/kg intraperitoneal behandelt. Die Schlafzeit der Tiere wurde registriert, die Durchschnittsschlaufdauer der einzelnen Gruppen wurde mit der Schlafzeit der Kontrolle verglichen und der Unterschied in Prozent ausgedrückt.

Die Ergebnisse der pharmakologischen Untersuchungen sind in der Tabelle I angegeben. Folgende Verbindungen wurden verwendet:

Verbindung A: 1-[4'-(Oxo)-1-[cyclohexyl]-3,4-di-[hydro]-6,7-di-[methoxy]-isochinolin

Verbindung B: 1-[{3'-(Methoxycarbonyl)-4'-(oxo)}-cyclohex-1'-yl]-3,4-di-[hydro]-6,7-di-[methoxy]-isochinolin

TABELLE I

| Verbindung | MET | FCS | ETA |
|---|---|---|---|
| A | | −30% | −48% |
| B | 40% | | −55% |

Aus der Tabelle ist ersichtlich, daß beide Verbindungen die alkoholische Schalfdauer beträchtlich abkürzen. Die Verbindung B zeigt darüber hinaus eine bedeutende krampfhemmende Wirkung, während die Verbindung A schmerzstillend wirkt.

Die erfindungsgemäßen 1-[Cyclohexyl]-3,4-di-[hydro]-isochinolinderivate, der allgemeinen Formel I, bei welchen X für Sauerstoff oder einen Rest der Formel IV, in welcher $R_4$ Wasserstoff bedeutet, steht und X einen Rest der allgemeinen Formel III darstellt sowie $R_1$ und $R_2$ unabhängig voneinander für Hydroxygruppen beziehungweise Alkoxyreste mit 1 bis 6 Kohlenstoffatomen stehen, sind auch Zwischenprodukte zur Herstellung der in der europäischen Offenlegungsschrift 161 478) beschriebenen neuen 1-[2'-(substituierten)-5',6',7',8'-Tetra-(hydro)-4'-(oxo)-chinazolin-6'-yl]-3,4-di-[hydro]-6,7-di-[substituierten)-isochinolinderivate [beziehungsweise als 4'-(Hydroxy)-Tautomere] beziehungsweise 1-[2'-(substituierten)-3',4',5',6',7',8'-Hexa-(hydro)-4'-(oxo)-chinazolin-6'-yl]-3,4-di-[hydro]-6,7-di-(substituierten]-isochinolinderivate der allgemeinen Formel

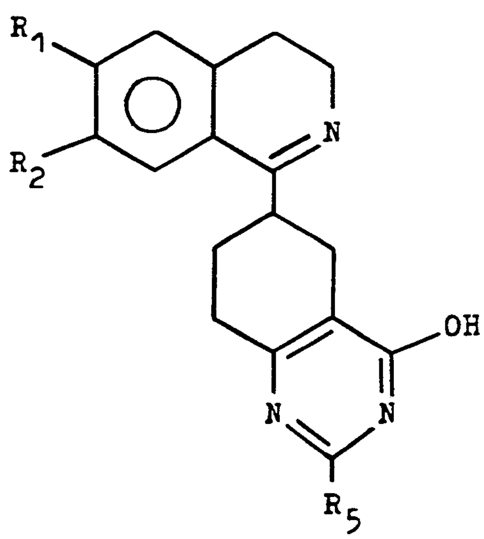

VIII

beziehungsweise

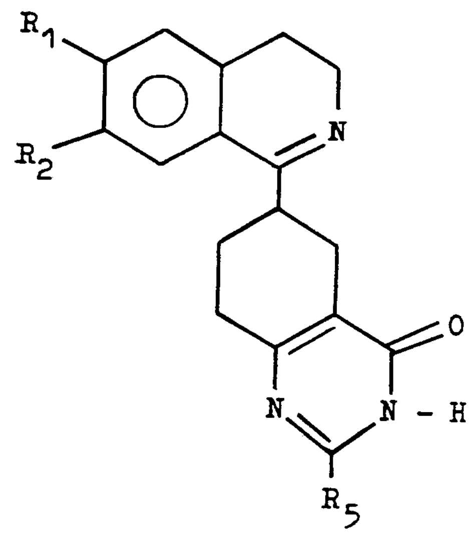

VIII,

worin

$R_1$ und $R_2$ unabhängig voneinander für Hydroxygruppen beziehungsweise Alkoxyreste mit 1 bis 6 Kohlenstoffatom(en) stehen und

$R_5$ einen Alkylrest mit 1 bis 6 Kohlenstoffatom(en), einen, gegebenenfalls substituierten, Phenylrest, einen, gegebenenfalls substituierten, Alkylphenylrest mit 1 bis 4 Kohlenstoffatom(en) im Alkylteil oder einen, gegebenenfalls substituierten, heterocyclischen Rest bedeutet,

sowie ihrer Säureadditionssalze und quaternären Salze.

Die Eigenschaften der genannten erfindungsgemäßen 1-[Cyclohexyl]-3,4-di-[hydro]-isochinolinderivate der allgemeinen Formel I sind die Ursache für die überlegenen pharmakologischen Wirkungen der aus ihnen herstellbaren Produkte 1-[2'-(substituierten)-5',6',7',8'-Tetra-(hydro)-4'-(oxo)-chinazolin-6'-yl]-3,4-di-[hydro]-6,7-di-[substituierten]-isochinolinderivate der allgemeinen Formel VIII beziehungsweise Säureadditionssalze beziehungsweise quaternären Salze derselben.

Die erfindungsgemäßen 1-[Cyclohexyl]-3,4-di-[hydro]-isochinolinderivate der allgemeinen Formel I, bei welchen X für Sauerstoff oder einen Rest der Formel IV, in welcher $R_4$ Wasserstoff bedeutet, steht und X einen Rest der allgemeinen Formel III darstellt sowie $R_1$ und $R_2$ unabhängig voneinander für Hydroxygruppen beziehungsweise Alkoxyreste mit 1 bis 6 Kohlenstoffatomen stehen, können in der Weise in die 1-[2'-(substituierten)-5',6',7',8'-Tetra-(hydro)-4'-(oxo)-chinazolin-6'-yl]-3,4-di-[hydro]-6,7-di-[substituierten]-isochinolinderivate der allgemeinen Formel VIII beziehungsweise ihre Säureadditionssalze und quaternären Salze überführt werden, daß die ersteren, gegebenenfalls nach ihrem Überführen in [andere] reaktionsfähige Derivate, mit Amidinen der allgemeinen Formel

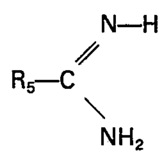

IX,

worin $R_5$ die oben angegebenen Bedeutungen hat, oder mit Salzen derselben mit anorganischen oder organischen Säuren in einem basischen Medium kondensiert werden,

worauf

gegebenenfalls in den erhaltenen 1 - [2' - (substituierten) - 5',6',7',8' - Tetra - (hydro) - 4' - (oxo) - chinazolin - 6' - yl] - 3,4 - di - [hydro] - 6,7 - di - [substituierten] - isochinolinderivaten der allgemeinen Formel VIII der beziehungsweise die Substituenten, für welche[n] $R_1$ und/oder $R_2$ und/oder $R_5$ steht beziehungsweise stehen, zu [einem] anderen Substituenten, welche[n] $R_1$ und/oder $R_2$ und/oder $R_5$ bedeuten kann beziehungsweise können, umgesetzt wird beziehungsweise werden und/oder

gegebenenfalls die erhaltenen 1 - [2' - (substituierten) - 5',6',7',8' - Tetra - (hydro) - 4' - (oxo) - chinazolin - 6' - yl] - 3,4 - di - [hydro] - 6,7 - di - [substituierten] - isochinolinderivate der allgemeinen Formel VIII in Säureaddiitionssalze oder quaternäre Salze überführt werden und/oder gegebenenfalls die erhaltenen Säureadditionssalze der 1 - [2' - (substituierten) - 5',6',7,8' - Tetra - (hydro) - 4' - (oxo) - chinazolin - 5' - yl] - 3,4 - di - [hydro] - 6,7 - di - [substituierten] - isochinolinderivate der allgemeinen Formel VIII in die freien 1-[2'-(substituierten)-5',6',7',8'-Tetra-(hydro)-4'-(oxo)-chinazolin-6'-yl]-3,4-[hydro]-6,7-di-[substituierten]-isochinolinderivate der allgemeinen Formel VIII oder in andere Säureadditionssalze oder quaternäre Salze überführt werden. Dabei werden vorteilhaft zur Herstellung des basischen Mediums Alkalimetallhydroxyde, -carbonate und/oder -alkoholate verwendet.

Vorteilhafte 1-[2'-(substituierte)-5',6',7',8'-Tetra-(hydro)-4'-(oxo)-chinazolin-6'-yl]-3,4-di-[hydro]-6,7-di-[substituierte]-isochinolinderivate der allgemeinen Formel VIII, in welche die genannten erfindungsgemäßen 1-[Cyclohexyl]-3,4-di-[hydro]-isochinolinderivate der allgemeinen Formel I überführt werden können, sind solche, bei welchen der beziehungsweise die Alkoxyrest(e) und/oder Alkylrest(e), für welche[n] $R_1$ und/oder $R_2$ und/oder $R_5$ stehen kann beziehungsweise können, [ein] solche[r] mit 1 bis 4 insbesondere 1 oder 2, Kohlenstoffatom(en) ist beziehungsweise sind.

Ferner sind vorteilhafte 1-[2'-(substituierte)-5',6',7',8'-Tetra-(hydro)-4'-(oxo)-chinazolin-6'-yl]-3,4-di-[hydro]-6,7-di-[substituierte]-isochinolinderivate der allgemeinen Formel VIII, in welche die genannten erfindungsgemäßen 1-[Cyclohexyl]-3,4-di-[hydro]-isochinolinderivate der allgemeinen Formel I überführt werden können, solche, bei welchen der Alkylphenylrest, für welchen $R_5$ stehen kann, ein solcher mit 1 oder 2, insbesondere 1, Kohlenstoffatom(en) im Alkylteil ist.

Weiterhin sind vorteilhafte 1-[2'-(substituierte)-5',6',7',8'-Tetra-(hydro)-4'-(oxo)-chinazolin-6'-yl]-3,4-di-[hydro]-6,7-di-[substituierte]-isochinolinderivate der allgemeinen Formel VIII, in welche die genannten erfindungsgemäßen 1-[Cyclohexyl]-3,4-di-[hydro]-isochinolinderivate der allgemeinen Formel I überführt werden können, solche, bei welchen der beziehungsweise die Substituent(en), durch welche[n] der Phenylrest oder Alkylphenylrest, für den $R_5$ stehen kann, substituiert sein kann, 1 oder mehr Halogenatom(e), wie Chlor, Fluor-, Brom- und/oder Jodatom(e), insbesondere Chloratom(e), Alkoxyrest(e) mit 1 bis 6, insbesondere 1 bis 4, ganz besonders 1 oder 2, vor allem 1, Kohlenstoffatom(en), Alklyrest(e) mit 1 bis 6, insbesondere 1 bis 4, ganz besonders 1 oder 2, vor allem 1, Kohlenstoffatom(en) und/oder Trihalogenmethylrest(e), insbesondere Trifluormethylrest(e) ist beziehungsweise sind.

Außerdem sind vorteilhafte 1-[2'-(substituierte)-5',6',7',8'-Tetra-(hydro)-4'-(oxo)-chinazolin-6'-yl]-3,4-di-[hydro]-6,7-di-[substituierte]-isochinolinderivate der allgemeinen Formel VIII, in welche die genannten erfindungsgemäßen 1-[Cyclohexyl]-3,4-di-[hydro]-isochinolinderivate der allgemeinen Formel I überführt werden können, solche, bei welchen der heterocyclische Rest, für den $R_5$ stehen kann, 5 bis 7-gliedrig, insbesondere 5- oder 6-gliedrig, ganz besonders 6-gliedrig, ist und als Heteroatom(e) 1 oder mehr Stickstoff-, Sauerstoff- und/oder Schwefelatom(e) aufweist, vor allem ein Pyridyl-, Piperidinyl-, Morpholinyl-, Piperazinyl-, 1,2,5,6-Tetrahydropyridinyl- oder Pyrrolidinylrest, ist.

Ferner sind vorteilhafte 1-[2'-(substituierte)-5',6',7',8'-Tetra-(hydro)-4'-(oxo)-chinazolin-6'-yl]-3,4-di-[hydro]-6,7-di-[substituierte]-isochinolinderivate der allgemeinen Formel VIII, in welche die genannten erfindungsgemäßen 1-[Cyclohexyl]-3,4-di-[hydro]-isochinolinderivate der allgemeinen Formel I überführt werden können, solche, bei welchen der beziehungsweise die Substituent(en), durch welche[n] der heterocyclische Rest, für den $R_5$ stehen kann, substituiert sein kann, 1 oder mehr, Alkylrest(e) mit 1 bis 4, insbesondere 1 oder 2, ganz besonders 1, Kohlenstoffatom(en), gegebenenfalls durch 1 oder mehr Halogenatom(e), wie Chlor-, Fluor-, Brom- und/oder Jodatom(e), insbesondere Chloratom(e), substituierte[r], Phenylrest(e), Hydroxygruppe(n), Carbamoylrest(e) und/oder weitere[r], vorteilhaft 5- bis 7-gliedrige[r], insbesondere 5- oder 6-gliedrige[r], ganz besonders 6-gliedrige[r], und als Heteroatom(e) 1 oder mehr Stickstoff-, Sauerstoff- und/oder Schwefelatom(e) aufweisende[r], heterocyclisher Rest, vor allem [ein] Pyridyl-, Piperidinyl-, Morpholinyl-, Piperazinyl-, 1,2,5,6-Tetrahydropyridinyl- oder Pyrrolidinylrest, ist beziehungsweise sind.

Bevorzugte 1 - [2' - (substituierte) - 5',6',7',8' - Tetra - (hydro) - 4' - (oxo) - chinazolin - 6' - yl] - 3,4 - di - [hydro] - 6,7 - di - [substituierte] - isochinolinderivate der allgemeinen Formel VIII, in welche die genannten erfindungsgemäßen 1 - [Cyclohexyl] - 3,4 - di - [hydro] - isochinolinderivate der allgemeninen Formel I überführt werden können, sind 1 - [2' - (Piperidin - 1'' - yl) - 5',6',7',8' - tetra - (hydro) - 4' - (oxo) - chinazolin - 6' - yl] - 3,4 - di - [hydro] - 6,7 - di - [methoxy] - isochinolin, 1 - [2' - (4'' - {p - <Chlor> - phenyl} - 4'' - {hydroxy} - piperidin - 1'' - yl) - 5',6',7',8' - tetra - (hydro) - 4' - (oxo) - chinazolin - 6' - yl] - 3,4 - di - [hydro] - 6,7 - di - [methoxy] - isochinolin, 1 - [2' - (4'' - {Piperidin - 1''' - yl} - 4'' - {carbamoyl} - piperidin - 1'' - yl) - 5',6',7',8' - tetra - (hydro) - 4' - (oxo) - chinazolin - 6' - yl] - 3,4 - di - [hydro] - 6,7 - di - [methoxy] - isochinolin, 1 - [2' - (3'',4'',5'' - Tri - {methoxy} - phenyl) - 5,'6',7',8' - tetra - (hydro) - 4' - (oxo) - chinazolin - 6' - yl] - 3,4 - di - [hydro] - 6,7 - di - [methoxy] - isochinolin, 1 - [2' - (1'',2'',5'',6'' - Tetra - {hydro} - 4'' - {phenyl} - pyrid - 1'' - yl) - 5',6',7',8' - tetra - (hydro) - 4' - (oxo) - chinazolin - 6' - yl] - 3,4 - di - [hydro] - 6,7 - di - [methoxy] - isochinolin, 1 - [2' - (4'' - {2''' - <Chlor> - phenyl} - piperazin - 1'' - yl) - 5',6',7',8' - tetra - (hydro) - 4' - (oxo) - chinazolin - 6' - yl] - 3,4 - di - [hydro] - 6,7 - di - [methoxy] - isochinolin, 1 - [2' - (3'' - <Methyl> - phenyl) - 5,'6',7',8' - tetra - (hydro) - 4' - (oxo) - chinazolin - 6' - yl] - 3,4 - di - [hydro] - 6,7 - di - [methoxy] - isochinolin, 1 - [2' - (Pyrid - 3'' - yl) - 5',6',7',8' - tetra - (hydro) - 4' - (oxo) - chinazolin - 6' - yl] - 3,4 - di - [hydro] - 6,7 - di - [methoxy] - isochinolin und 1 - [2' - (Pyrrolidin - 1'' - yl) - 5',6',7',8' - tetra - (hydro) - 4' - (oxo) - chinazolin - 6' - yl)] - 3,4 - di - [hydro] - 6,7 - di - [methoxy] - isochinolin [beziehungsweise jeweils als 4' - (Hydroxy) - Tautomer] sowie ihre Säureadditionssalze und quaternären Salze. Auch in den obigen Verbindungen ist der Ausdruck "-5',6',7',8'-tetra-(hydro)-" als wahlweise auch "-3',4',5',6',7',8'-hexa-(hydro)-" umfassend zu verstehen, da die obigen Verbindungen tautomer (Keto/Enol-Tautomerie) sind.

Die Erfindung wird an Hand der folgenden Ausführungsbeispiele näher erläutert.

Beispiel 1

1-[{3'-(Methoxycarbonyl)-4'-(oxo)}-cyclohex-1'-yl]-3,4-di-[hydro]-6,7-di-[methoxy]-isochinolin beziehungsweise 1-[{3'-(Äthoxycarbonyl)-4'-(oxo)}-cyclohex-1'-yl]-3,4-di-[hydro]-6,7-di-[methoxy]-isochinolin

Das wie unten beschrieben erhaltene Öl von 4-[3',4'-Di-(hydro)-(2'H)-6',7'-di-(methoxy)-isochinolin-1'-yl]-heptan-1,7-dicarbonsäure-dimethylester wird in 500 ml Toluol gelöst. Die Lösung wird zu in einem Kolben von 1 Liter Volumen befindlichem, zur Trockne eingedampftem Natriummethylat gegeben, das aus 30 g Natrium hergestellt wurde. Das Natriummethylat wird im Rotationsverdampfer unter schwachem Erwärem gelost. Noch wahrend des Lösungsvorganges beginnt sich das Natriumsalz abzuscheiden. Parallel dazu wird das Gerät langsam unter verminderten Druck gesetzt und ein Teil des Lösungsmittels bei etwa 30 mm Hg abdestilliert. Das fehlende Toluol wird ersetzt. Dann wird das Gemisch auf dem Ölbad 3 Stunden lang unter Rückfluß gekocht. Abdestillieren und erneuter Zusatz des Lösungsmittels werden wiederholt, anschließend wird weitere 6 Stunden lang unter Rückfluß gekocht. Dann wird das Toluol zum großen Teil abdestilliert. Nach dem Abkühlen wird das Gemisch mit 25 %iger Salzsäure angesäuert, wodurch das Produkt in Lösung geht. In einem Scheidetrichter werden die Phasen voneinander getrennt. Die wäßrige Phase wird mit Aktivkohle geklärt, dann filtriert und mit gesättigter Kaliumcarbonatlösung alkalisch gemacht. Die ausgefallenen Kristalle werden aus der gekühlten Lösung abfiltriert, nit wenig Wasser und dann mit Äther gewaschen und schließlich getrocknet. Ausbeute: 76 g (91%)

In analoger Weise kann auch das 1 - [{3' - (Methoxycarbonyl) - 4' - (oxo)} - cyclohex - 1' - yl] - 3,4 - di - [hydro] - 6,7 - di - [äthoxy] - isochinolin beziehungsweise 1 - [{3' - (Äthoxycarbonyl) - 4' - (oxo)} - cyclohex - 1' - yl] - 3,4 - di - [hydro] - di - [äthoxy] - isochinolin hergestellt werden.

Zur Herstellung der Hydrochloride wird die jeweilige Base in absolutem Äthanol gelöst und mit der wasserfreien äthanolischen Lösung eines eineinhalbfachen Überschusses von Chlorwasserstoffgas versetzt. Die Lösung wird eingedampft, der Rückstand in wenig absolutem Äthanol gelöst und das Hydrochlorid durch Zusatz von Äther ausgefällt.

Die physikalischen Daten der gemäß Beispiel 1 hergestellten Verbindungen sind in der Tabelle 1 zusammengestellt.

Der als Ausgangssubstanz verwendete 4-[3',4'-Di-(hydro)-(2'H)-6',7'-di-(methoxy)-isochinolin-1'-yl]-heptan-1,7-dicarbonsäure-dimethyl-beziehungsweise-diäthylester ist wie folgt beschrieben erhalten worden.

4 - [3',4' - Di - (hydro) - (2'H) - 6',7' - di - (methoxy) - isochinolin - 1' - yl] - heptan - 1,7 - dicarbonsäure - dimethyl - beziehungsweise - diäthylester [Verbindung der allgemeinen Formel VI].

82 g (0,4 Mol) 1-(Methyl)-3,4-di-(hydro)-6,7-di-(methoxy)-isochinolin werden in 100 ml Benzol gelöst. Die Lösung wird mit 160 g (etwa 1,6 Mol) Methyl- beziehungsweise Äthylacrylat versetzt und dann etwa 24 Stunden lang unter Rückfluß gekocht. Das Voranschreiten der Additionsreaktion wird dünnschichtchromatographisch verfolgt. Der Oberschuß des Acrylats und das Benzol werden abdestilliert. Das zurückbleibende braune Öl kann ohne weitere Reinigung für die nachfolgende Umsetzung verwendet werden.

In analoger Weise können auch 4 - [3',4' - Di - (hydro) - (2'H) - 6,',7' - di - (äthoxy) - isochinolin - 1' - yl] - heptan - 1,7 - dicarbonsäuredimethyl - und - diäthylester hergestellt werden.

TABELLE II

1-[{3'-Methoxy- beziehungsweise -Äthoxycarbonyl)-4'-(oxo)}-cyclohex-1'-yl]-3,4-di-[hydro]-6,7-di-[methoxy]-isochinoline

(Verbindungen der allgemeinen Formel

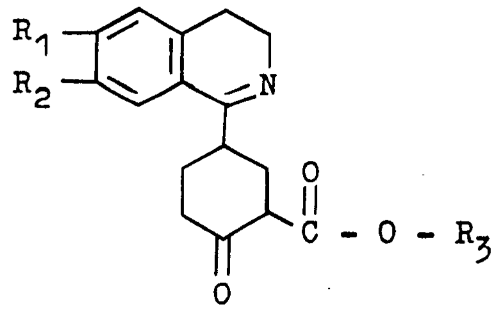

IA)

| $R_1 = R_2$ | $R_3$ | HX | Summenformel Molgewicht | Schmp. °C Lsgsm. | Analyse, ber. | % gef. | Ausbeute % |
|---|---|---|---|---|---|---|---|
| $-OCH_3$ | $-CH_3$ | | $C_{19}H_{23}NO_5$ 335,39 | 125—126 Methanol/ Äther | C 68,04<br>H 6,91<br>N 4,14 | 68,25<br>7,18<br>4,36 | 91 |
| $-OCH_3$ | $-CH_3$ | HCl | $C_{19}H_{24}NO_5Cl$ 381,85 | 200 Methanol/ Äther | C 59,76<br>H 6,33<br>N 3,65<br>Cl 9,28 | 59,50<br>6,81<br>3,75<br>9,49 | |
| $-OCH_3$ | $-C_2H_5$ | | $C_{20}H_{25}NO_5$ 359,42 | 103—105 Äther | C 66,83<br>H 7,01<br>N 3,89 | 67,28<br>7,08<br>4,01 | 83 |
| $-OCH_3$ | $-C_2H_5$ | HCl | $C_{20}H_{26}NO_5Cl$ 395,88 | 213—214 Äthanol | C 60,67<br>H 6,61 | 60,75<br>6,25 | |

Beispiel 2

1-[4'-(Oxo)-cyclohexyl]-3,4-di-[hydro]-6,7-di-[methoxy]-isochinolin

16,7 g (0,05 Mol) 1-[{3'-(Methoxycarbonyl)-4'-(oxo)}-cyclohex-1'-yl]-3,4-di[hydro]-6,7-di-[methoxy]-isochinolin werden in 100 ml 4 n Salzsäure gelöst. Die Lösung wird eine Stunde lang, bis zur Beendigung der Kohlhendioxydentwicklung, schwach am Sieden gehalten. Dann wird die Lösung zur Trockne eingedampft, der Rückstand in Wasser geölst, die Lösung mit Ammoniumhydroxyd alkalisch gemacht, die ausgefallenen Kristalle abgesaugt und das Äther umkristallisiert. Ausbeute: 74%, Schmp.: 103—105°C.

Elementaranalyse für: $C_{17}H_{21}NO_3$ (M = 287,36)
berechnet, %: C 71,05, H 7,36, N 4,87;
gefunden, %: C 70,82, H 7,29, N 4,90.

Aus der Verbindung wird mit einem Überschuß an methanolischer Salzsäure das Hydrochlorid bereitet, das bei 207—208°C schmilzt (Methanol/Aceton).

Elementaranalyse für $C_{17}H_{22}NO_3Cl$ (M = 323,87)
berechnet, %: C 63,05, H 6,84, N 4,32, Cl 10,34;
gefunden, %: C 62,67, H 6,21, N 4,45 Cl 10,53.

Beispiel 3

1-[{3'-(Cyano)-4'-(imino)}-cyclohex-1'-yl-]-3,4-di-[hydro]-6,7-di-[methoxy]-isochinolin

12,4 g (0,04 Mol) des wie unten beschrieben hergestellten Dinitrils werden in 200 ml Toluol gelöst. Die Lösung wird mit 5,4 g (0,1 Mol) Natriummethylat versetzt und dann etwa 50 Stunden lang unter Rückfluß gekocht. Der Verlauf der Reaktion wird dünnschichtchromatographisch verfolgt. Nach Beendigung der Umsetzung wird das abgekühlte Reaktionsgemisch mit verdünnter Salzsäure angesäuert, und die wäßrige Phase wird abgetrennt. Die wäßrige Phase wird dann mit Sodalösung alkalisch gemacht und mit Chloroform mehrmals extrahiert. Die vereinigten organischen Phasen werden über wasserfreiem Natriumsulfat getrocknet und dann im Vakuum eingedampft. Aus dem öligen Rückstand wird mit äthanolischer Salzsäure

das Hydrochlorid hergestellt und dann aus Äthanol umkristallisiert. Ausbeute: etwa 30%, Schmp.: ab 255°C unter Zersetzung.

Das als Ausgangssubstanz verwendete 4-[3',4'-Di-(hydro)-(2'H)-6',7'-di-(methoxy)-isochinolin-1'-yl]-heptan-1,7-di-carbonsäurenitril ist wie folgt beschrieben erhalten worden.

4 - [3',4' - Di - (hydro) - (2'H) - 6',7' - di - (methoxy) - isochinolin - 1' - yl] - heptan - 1,7 - di-carbonsäurenitril [Verbindung der allgemeinen Formel VI].

23.3 g (0,1 Mol) 1-(Methyl)-3,4-di-(hydro)-6,7-di-(methoxy)-isochinolin werden in einem Gemisch aus 100 ml Benzol und 26,5 ml (0,04 Mol) Acrylnitril etwa 50 Stunden lang unter Rückfluß gekocht. Der Reaktionsverlauf wird dünnschichtchromatographisch verfolgt. Nach Beendigung der Umsetzung werden Acrylnitril und Benzol abdestilliert, und der Rückstand wird aus Methanol umkristallisiert. Nach nochmaligem Kristallisieren liegt der Schmelzpunkt bei 106°C. Das Hydrochlorid wird mit äthanolischer Salzsäure hergestellt und aus Äthanol umkristallisiert. Schmelzpunkt: 168—169°C. Ausbeute: etwa 60%.

Beispiel 4

1-[Cyclohex-1'-yl-4'-(oxim)]-3,4-di-[hydro]-6,7-di-[methoxy]-isochinolin

0,5 g 1-[4'-Oxo)-cyclohexyl]-3,4-di-[hydro]-6,7-di-[methoxy]-isochinolin (X = O, Z = $CH_2$—) und 0,5 g Hydroxylaminhydrochlorid werden in 5 ml Äthanol suspendiert. Die Suspension wird mit 0,5 ml Pyridin versetzt und dann 60 Minuten lang unter Rückfluß gekocht. Dann wird das Reaktionsgemisch eingedampft, mit Wasser verdünnt und gekühlt. Die ausgefallenen Kristalle werden abfiltriert, gewaschen und getrocknet. Die Titelverbindung schmilzt bei 192°C. Ausbeute: 51%.

Elementaranalyse:

berechnet, %: C 67,52, H 7,33, N 9,26;
gefunden, %: C 68,49, H 7,41, N 10,11.

Beispiel 5

1-[{3'-(Methoxycarbonyl)-4'-(imino)}-cyclohex-1'-yl]-3,4-di-[hydro]-6,7-di-[methoxy]-isochinolin

6,68 g (0,02 Mol) 1-[{3'-(Methoxycarbonyl)-4-'-(oxo)}-cyclohex-1'-yl]-3,4-di-[hydro]-6,7-di-[methoxy)-isochinolin werden unter leichtem Erwärmen in 100 ml 28 %igem ammoniakalischem Methanol gelöst. Die Lösung wird bei Raumtemperatur zwei Tage lang stehengelassen und dann im Wasserstrahlvakuum (30 mm Hg) auf die Hälfte ihres Volumens eingeengt. Die beim Kühlen ausfallenden Kristalle werden abfiltriert. Man erhält die Titelverbindung in einer Menge von 5,5 g (80%). Schmp.: 114—115°C.

Elementaranalyse für $C_{19}H_{23}N_2O_4$ (M = 343,40)

berechnet, %: C 66,45, H 6,75, N 8,50;
gefunden, %: C 66,15, H 6,92, N 8,26.

**Patentansprüche für die Vetragsstaaten: BE CH DE FR GB IT LI NL SE**

1. 1-[Cyclohexyl]-3,4-di-[hydro]-isochinolinderivate der allgemeinen Formel

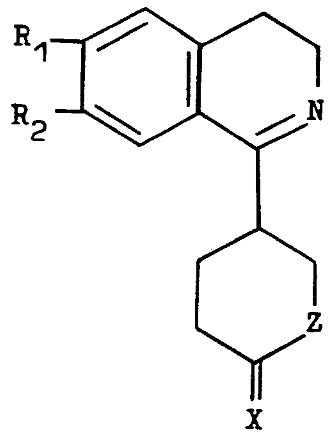

I,

worin

$R_1$ und $R_2$ unabhängig voneinander für Wasserstoff, Hydroxygruppen beziehungsweise Alkoxyreste mit 1 bis 6 Kohlenstoffatom(en) stehen und entweder

X Sauerstoff bedeutet und

Z einen Rest der Formel

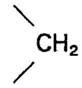

II,

oder einen Rest der allgemeinen Formel

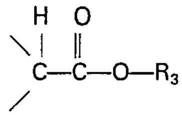

III,

in welchletzterer $R_3$ für Wasserstoff oder einen Alkylrest mit 1 bis 6 Kohlenstoffatom(en) steht, darstellt oder

X einen Rest der allgemeinen Formel

$$=N—R_4 \qquad \text{IV,}$$

in welchletzterer $R_4$ für Wasserstoff oder eine Hydroxygruppe steht, bedeutet und

Z einen Rest der Formel

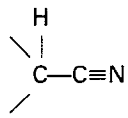

V,

einen Rest der Formel

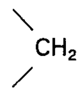

II

oder einen Rest der allgemeinen Formel

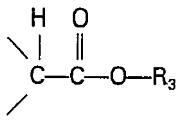

III,

in welchletzterer $R_3$ für Wasserstoff oder einen Alkylrest mit 1 bis 6 Kohlenstoffatom(en) steht, darstellt, sowie ihre Säureadditionssalze.

2. 1-[Cyclohexyl]-3,4-di-[hydro]-isochinolinderivate nach Anspruch 1, dadurch gekennzeichnet, daß die Alkoxyreste, für welche $R_1$ und/oder $R_2$ stehen können, solche mit 1 bis 4 Kohlenstoffatomen sind.

3. 1-[Cyclohexyl]-3,4-di-[hydro]-isochinolinderivate nach Anspruch 2, dadurch gekennzeichnet, daß die Alkoxyreste, für welche $R_1$ und/oder $R_2$ stehen können, solche mit 1 oder 2 Kohlenstoffatomen sind.

4. 1-[Cyclohexyl]-3,4-di-[hydro]-isochinolinderivate nach Anspruch 1 oder 2, dadurch gekennzeichnet, daß die Alkylrest, für den $R_3$ stehen kann, ein solcher mit 1 bis 4 Kohlenstoffatomen ist.

5. 1-[Cyclohexyl]-3,4-di-[hydro]-isochinolinderivate nach Anspruch 4, dadurch gekennzeichnet, daß der Alkylrest, für welchen $R_3$ stehen kann, ein solcher mit 1 oder 2 Kohlenstoffatomen ist.

6. Verbindungen gemäß Anspruch 1, dadurch gekennzeichnet, daß sie
1 - [4' - (Oxo) - cyclohexyl] - 3,4 - di - [hydro] - 6,7 - di - [methoxy] - isochinolin, 1 - [{3' - (Methoxycarbonyl) - 4' - (oxo)} - cyclohex - 1' - yl] - 3,4 - di - [hydro] - 6,7 - di - [methoxy] - isochinolin, 1-[{3' - (Äthoxycarbonyl) - 4' - (oxo)} - cyclohex - 1' - yl] - 3,4 - di - [hydro] - 6,7 - di - [methoxy] - isochinolin, 1 - [{3' - (Cyano) - 4' - (imino)} - cyclohex - 1' - yl] - 3,4 - di - [hydro] - 6,7 - di - [methoxy] - isochinolin, 1 - [Cyclohex - 1' - yl - 4' - (oxim)] - 3,4 - di - [hydro] - 6,7 - di - [methoxy] - isochinolin und 1 - [{3' - (Methoxycarbonyl) - 4' - (imino)} - cyclohex - 1' - yl] - 3,4 - di - [hydro] - 6,7 - di - [methoxy] - isochinolin sowie ihre Hydrochloride sind.

7. Verfahren zur Herstellung der Verbindungen nach Anspruch 1 bis 6, dadurch gekennzeichnet, daß man in an sich bekannter Weise

a) zur Herstellung von 1-[Cyclohexyl)-3,4-di-[hydro]-isochinolinderivaten der allgemeinen Formel I, bei welchen X für Sauerstoff steht und Z einen Rest der allgemeinen Formel III, in welcher $R_3$ einen Alkylrest mit 1 bis 6 Kohlenstoffatom(en) darstellt, bedeutet sowie $R_1$ und $R_2$ dieselben Bedeutugen wie in den

Ansprüchen 1 bis 3 haben, 4-[3',4'-Di-(hydro)-(2'H)-isochinolin-1'-yl]-heptan-1,7-di-[carbonsäure]-derivate der allgemeinen Formel

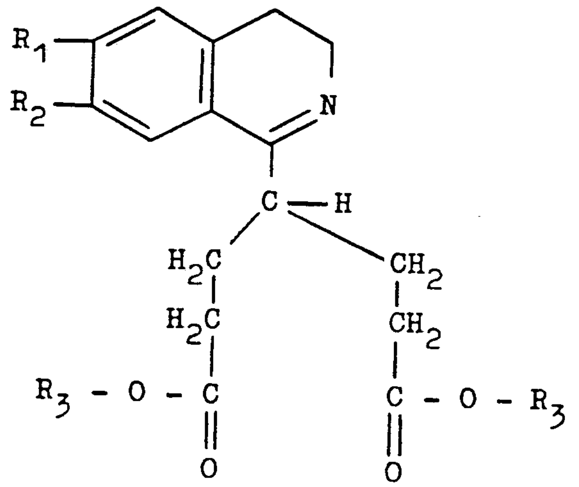

VI,

worin $R_1$, $R_2$ und $R_3$ dieselben Bedeutungen wie in den Ansprüchen 1 bis 5 haben, in Gegenwart von Alkalimetallen, Alkalimetallalkoholaten oder Alkalimetallamiden cyclisiert oder

b) zur Herstellung von 1-[Cyclohexyl]-3,4-di-[hydro]-isochinolinderivaten der allgemeinen Formel I, bei welchen X für einen Rest der allgemeinen Formel IV, in welcher $R_4$ Wasserstoff bedeutet, steht und Z einen Rest der Formel V darstellt, sowie $R_1$ und $R_2$ dieselben Bedeutungen wie in den Ansprüchen 1 bis 3 haben, 4-[3',4'-Di-(hydro)-(2'H)-isochinolin-1'-yl]-heptan-1,7-di-[carbonsäurenitril]-derivate der allgemeinen Formel

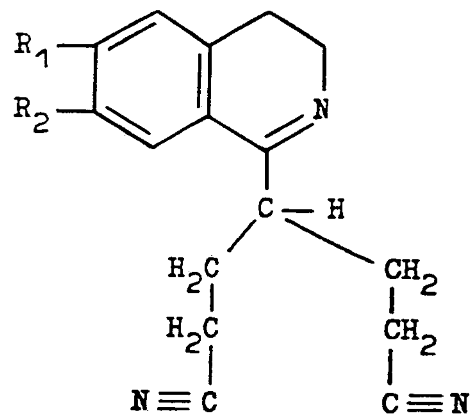

VII,

worin $R_1$ und $R_2$ dieselben Bedeutungen wie in den Ansprüchen 1 bis 3 haben, in Gegenwart von Alkalimetallen, Alkalimetallalkoholaten oder Alkalimetallamiden cyclisiert, worauf man

gegebenenfalls nach der Variante a) erhaltene 1-[Cyclohexyl]-3,4-di-[hydro]-isochinolinderivate der allgemeinen Formel I, bei welchen Z, X, $R_1$, $R_2$ und $R_3$ die bei der Variante a) angegebenen Bedeutungen haben, zu den entsprechenden 1-[Cyclohexyl]-3,4-di-[hydro]-isochinolinderivaten der allgemeinen Formel I, bei welchen Z für einen Rest der allgemeinen Formel III, in welcher $R_3$ Wasserstoff bedeutet, steht oder einen Rest der Formel II darstellt, hydrolysiert und/oder decarboxyliert und/oder

gegebenenfalls gegebenenfalls erhaltene 1-[Cyclohexyl]-3,4-di-[hydro]-isochinolinderivate der allgemeinen Formel I, bei welchen X für Sauerstoff steht und Z, $R_1$, $R_2$ und $R_3$ dieselben Bedeutungen wie in den Ansprüchen 1 bis 5 haben, durch Umsetzen mit Hydroxylamin beziehungsweise Ammoniak zu den entsprechenden 1-[Cyclohexyl]-3,4-di-[hydro]-isochinolinderivaten der allgemeinen Formel I, bei welchen X einen Rest der allgemeinen Formel IV bedeutet, umsetzt und/oder

gegebenenfalls in 1-[Cyclohexyl]-3,4-di-[hydro]-isochinolinderivaten der allgemeinen Formel I den beziehungsweise die Substituenten, für welche[n] $R_1$ und/oder $R_2$ steht beziehungsweise stehen, zu [einem] anderen Substituenten, welche[n] $R_1$ und/oder $R_2$ bedeuten kann, umsetzt und/oder

gegebenenfalls die erhaltenen 1-[Cyclohexyl]-3,4-di-[hydro]-isochinolinderivate der allgemeinen Formel I in Säureadditionssalze überführt beziehungsweise gegebenenfalls die erhaltenen Säureadditionssalze der 1-[Cyclohexyl]-3,4-di-[hydro]-isochinolinderivate der allgemeinen Formel I in die freien 1-[Cyclohexyl]-3,4-di-[hydro]-isochinolinderivate der allgemeinen Formel I oder in andere Säureadditionssalze überführt.

8. Arnzeimittel, gekennzeichnet durch einen Gehalt an 1 oder mehr Verbindung(en) nach den Ansprüchen 1 bis 6 als Wirkstoff(en), zweckmässigerweise zusammen mit 1 oder mehr üblichen pharmazeutischen Träger-, Streck- und/oder Hilfsstoff(en).

**Patentansprüche für den Vertragsstaat: AT**

1. Verfahren zur Herstellung von 1-[Cyclohexyl]-3,4-di-[hydro]-isochinolinderivaten der allgemeinen Formel

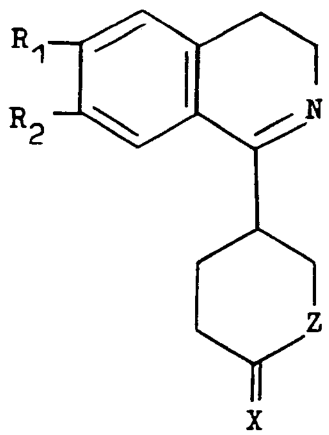

I,

worin

$R_1$ und $R_2$ unabhängig voneinander für Wasserstoff, Hydroxygruppen beziehungsweise Alkoxyreste mit 1 bis 6 Kohlenstoffatom(en) stehen und entweder

X Sauerstoff bedeutet und

Z einen Rest der Formel

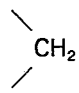

II,

oder einen Rest der allgemeinen Formel

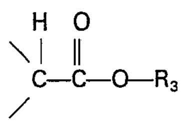

III,

in welchletzterer $R_3$ für Wasserstoff oder einen Alkylrest mit 1 bis 6 Kohlenstoffatom(en) steht, darstellt oder

X einen Rest der allgemeinen Formel

$$=N—R_4$$ IV,

in welchletzterer $R_4$ für Wasserstoff oder eine Hydroxygruppe steht, bedeutet und

Z einen Rest der Formel

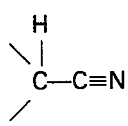

V,

einen Rest der Formel

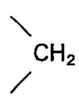

II,

oder einen Rest der allgemeinen Formel

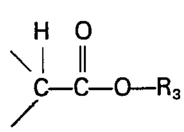

III,

in welchletzterer $R_3$ für Wasserstoff oder einen Alkylrest mit 1 bis 6 Kohlenstoffatom(en) steht, darstellt, sowie ihre Säureadditionssalze, dadurch gekennzeichnet, daß man in an sich bekannter Weise

a) zur Herstellung von 1-[Cyclohexyl)-3,4-di-[hydro]-isochinolinderivaten der allgemeinen Formel I, bei welchen X für Sauerstoff steht und Z einen Rest der allgemeinen Formel III, in welcher $R_3$ einen Alkylrest mit 1 bis 6 Kohlenstoffatom(en) darstellt, bedeutet sowie $R_1$ und $R_2$ dieselben Bedeutungen wie in den Ansprüchen 1 bis 3 haben, 4-[3',4'-Di-(hydro)-(2'H)-isochinolin-1'-yl]-heptan-1,7-di-[carbonsäure]-derivate der allgemeinen Formel

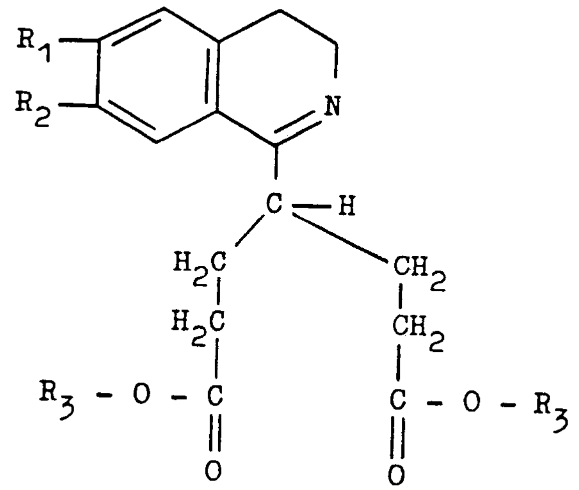

VI,

worin $R_1$, $R_2$ und $R_3$ dieselben Bedeutungen wie in den Ansprüchen 1 bis 5 haben, in Gegenwart von Alkalimetallen, Alkalimetallalkoholaten oder Alkalimetallamiden cyclisiert oder

b) zur Herstellung von 1-[Cyclohexyl]-3,4-di-[hydro]-isochinolinderivaten der allgemeinen Formel I, bei welchen X für einen Rest der allgemeinen Formel IV, in welcher $R_4$ Wasserstoff bedeutet, steht und Z einen Rest der Formel V darstellt, sowie $R_1$ und $R_2$ dieselben Bedeutungen wie in den Ansprüchen 1 bis 3 haben, 4-[3',4'-Di-(hydro)-(2'H)-isochinolin-1'-yl]-heptan-1,7-di-[carbonsäurenitril]-derivate der allgemeinen Formel

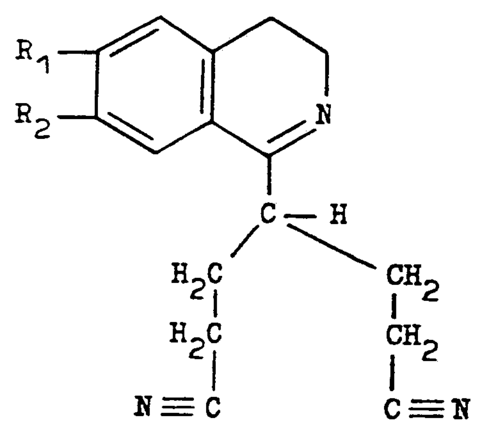

VII,

worin $R_1$ und $R_2$ dieselben Bedeutungen wie in den Ansprüchen 1 bis 3 haben, in Gegenwart von Alkalimetallen, Alkalimetallalkoholaten oder Alkalimetallamiden cyclisiert, worauf man

gegebenenfalls nach der Variante a) erhaltene 1-[Cyclohexyl]-3,4-di-[hydro]-isochinolinderivate der allgemeinen Formel I, bei welchen Z, X, $R_1$, $R_2$ und $R_3$ die bei der Variante a) angegebenen Bedeutungen haben, zu den entsprechenden 1-[Cyclohexyl]-3,4-di-[hydro]-isochinolinderivaten der allgemeinen Formel I, bei welchen Z für einen Rest der allgemeinen Formel III, in welcher $R_3$ Wasserstoff bedeutet, steht oder einen Rest der Formel II darstellt, hydrolysiert und/oder decarboxyliert und/oder

gegebenenfalls gegebenenfalls erhaltene 1-[Cyclohexyl]-3,4-di-[hydro]-isochinolinderivate der allgemeinen Formel I, bei welchen X für Sauerstoff steht und Z, $R_1$, $R_2$ und $R_3$ dieselben Bedeutungen wie in den Ansprüchen 1 bis 5 haben, durch Umsetzen mit Hydroxylamin beziehungsweise Ammoniak zu den entsprechenden 1-[Cyclohexyl]-3,4-di-[hydro]-isochinolinderivaten der allgemeinen Formel I, bei welchen X einen Rest der allgemeinen Formel IV bedeutet, umsetzt und/oder

gegebenenfalls in 1-[Cyclohexyl]-3,4-di-[hydro]-isochinolinderivaten der allgemeinen Formel I den beziehungsweise die Substituenten, für welche[n] $R_1$ und/oder $R_2$ steht beziehungsweise stehen, zu [einem] anderen Substituenten, welche[n] $R_1$ und/oder $R_2$ bedeuten kann, umgesetzt und/oder

gegebenenfalls die erhaltenen 1-[Cyclohexyl]-3,4-di-[hydro]-isochinolinderivate der allgemeinen Formel I in Säureadditionssalze überführt beziehungsweise gegebenenfalls die erhaltenen Säureadditionssalze der 1-[Cyclohexyl]-3,4-di-[hydro]-isochinolinderivate der allgemeinen Formel I in die freien 1-[Cyclohexyl]-3,4-di-[hydro]-isochinolinderivate der allgemeinen Formel I oder in andere Säureadditionssalze überführt.

2. Verfahren nach Anspruch 1, dadurch gekennzeichnet, daß solche 1-[Cyclohexyl]-3,4-di-[hydro]-isochinolinderivate hergestellt werden, bei welchen die Alkoxyreste, für welche $R_1$ und/oder $R_2$ stehen können, solche mit 1 bis 4 Kohlenstoffatomen sind.

3. Verfahren nach Anspruch 2, dadurch gekennzeichnet, daß solche 1-[Cyclohexyl]-3,4-di-[hydro]-isochinolinderivate hergestellt werden, bei welchen die Alkoxyreste, für welche $R_1$ und/oder $R_2$ stehen können, solche mit 1 oder 2 Kohlenstoffatomen sind.

4. Verfahren nach Anspruch 1 oder 2, dadurch gekennzeichnet, daß solche 1-[Cyclohexyl]-3,4-di-[hydro]-isochinolinderivate hergestellt werden, bei welchen der Alkylrest, für den $R_3$ stehen kann, ein solcher mit 1 bis 4 Kohlenstoffatomen ist.

5. Verfahren nach Anspruch 4, dadurch gekennzeichnet, daß solche 1-[Cyclohexyl]-3,4-di-[hydro]-isochinolinderivate hergestellt werden, bei welchen der Alkylrest, für welchen $R_3$ stehen kann, ein solcher mit 1 oder 2 Kohlenstoffatomen ist.

6. Verfahren nach Anspruch 1, dadurch gekennzeichnet, daß 1 - [4' - (Oxo) - cyclohexyl] - 3,4 - di - [hydro] - 6,7 - di - [methoxy] - isochinolin, 1 - [{3' - (Methoxycarbonyl) - 4' - (oxo)} - cyclohex - 1' - yl] - 3,4 - di - [hydro] - 6,7 - di - [methoxy] - isochinolin, 1-[{3' - (Äthoxycarbonyl) - 4' - (oxo)} - cyclohex - 1' - yl] - 3,4 - di - [hydro] - 6,7 - di - [methoxy] - isochinolin, 1 - [{3' - (Cyano) - 4' - (imino)} - cyclohex - 1' - yl] - 3,4 - di - [hydro] - 6,7 - di - [methoxy] - isochinolin, 1 - [Cyclohex - 1' - yl - 4' - (oxim)] - 3,4 - di - [hydro] - 6,7 - di - [methoxy] - isochinolin, und 1 - [{3' - (methoxycarbonyl) - 4' - (imino)} - cyclohex - 1' - yl] - 3,4 - di - [hydro] - 6,7 - di - [methoxy] - isochinolin sowie ihre Hydrochloride hergestellt werden.

**Revendications pour les Etats contractants: BE CH DE FR GB IT LI NL SE**

1. Dérivés de la 1-(cyclohexyl)-3,4-di(hydro)-isoquinoléine de formule générale I:

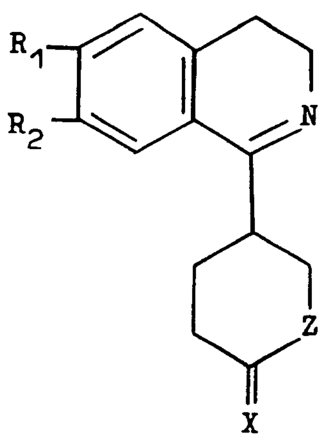

I,

dans laquelle $R_1$ et $R_2$, indépendants l'un de l'autre, représentent l'hydrogène, des groupes hydroxy, ou alkoxy de 1 à 6 atomes de carbone, et ou bien X désigne l'oxygène et Z un reste de formule II

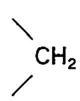

II,

ou un reste de formule III:

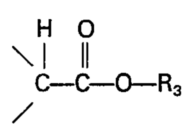

III,

dans lequel $R_3$ représente l'hydrogène, ou un alkyle de 1 à atomes de carbone, ou bien X signifie un reste de formule générale IV:

$$=N—R_4 \qquad IV$$

dans lequel $R_4$ est un atome d'hydrogène ou un groupe hydroxy et Z représente un reste de formule V:

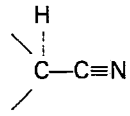

V,

un reste de formule II:

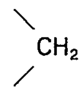

II,

ou un reste de formule générale III:

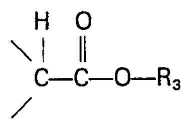

III,

dans lequel $R_3$ désigne un atome d'hydrogène ou un reste alkyle de 1 à 6 atomes de carbon, ainsi que leurs sels acides d'addition.

2. Dérivés de la 1-(cyclohexyl)-3,4-di(hydro)-isoquinoléine selon la revendication 1, caractérisés en ce que les restes alkoxy, représentant $R_1$ et/ou $R_2$, sont ceux comportant 1 à 4 atomes de carbone.

3. Dérivés de la 1-(cyclohexyl)-3,4-di(hydro)-isoquinoléine, selon la revendication 2, caractérisés en ce que les restes alkoxy, représentant $R_1$ et/ou $R_2$, sont ceux comportant 1 ou 2 atomes de carbone.

4. Dérivés de la 1-(cyclohexyl)-3,4-di(hydro)-isoquinoléine, selon la revendication 1 ou 2, caractérisé en ce que le reste alkyle, représentant $R_3$, comportant 1 à 4 atomes de carbone.

5. Dérivés de la 1-(cyclohexyl)-3,4-di(hydro)-isoquinoléine, selon la revendication 4, caractérisés en ce que le reste alkyle, représentant $R_3$, comporte 1 à 2 atomes de carbone.

6. Composés selon la revendication 1, caractérisés en ce qu'ils sont constitués par:
1-[4'(oxo)-cyclohéxyl]-3,4-di-[hydro]-6,7-di-[méthoxy]-isoquinoléine,
1-[{3'-(méthoxycarbonyl)-4'-(oxo)}-cyclohex-1'-yl]-3,4-di-[hydro]-6,7-di-[méthoxy]-isoquinoléine,
1-[{3'-(éthoxycarbonyl)-4'-(oxo)}-cyclohex-1'-yl]-4,6-di-[hydro]-6,7-di-[méthoxy]-isoquinoléine,
1-[{3'-(cyano)-4'-(imino)}-cyclohex-1'-yl]-3,4-di-[hydro]-6,7-di-[méthoxy]-isoquinoléine,
1-[cyclohex-1'-yl-4'-(oxim)]-3,4-di-[hydro]-6,7-di-[méthoxy]-isoquinoléine, et
1-[{3'-(méthoxycarbonyl)-4'-(imino)}-cyclohex-1'-yl]-3,4-di-[hydro]-6,7-di-[méthoxy]-isoquinoléine,
ainsi que leurs hydrochlorures.

Procédé de préparation des composés selon les revendications 1 à 6, caractérisé en ce que, de manière connue,

a) pour préparer les dérivés les dérivés 1-(cyclohexyl)-3,4-di-(hydro)-isoquinoléine de formule générale I dans lesquels X représente l'oxygène et Z un reste de formule générale II dans lequel $R_3$ est un reste alkyle de 1 à 6 atomes de carbone, $R_1$ et $R_2$ ayant les significations données dans les revendications 1 à 3, on cyclise le dérivé 4-3',4'-di-(hydro)-(2'H)-isoquinoléine-1'-yl-heptane-1,7-diacide carbonique de formule générale VI:

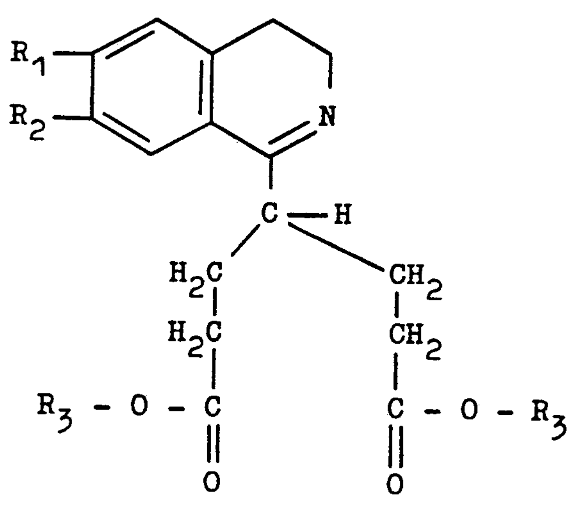

VI,

dans laquelle $R_1$ et $R_2$ ont la signification indiquée dans les revendications 1 à 5, en présence de métaux alcalins, d'alcoolates de métaux alcalins du d'amides de métaux alcalins, ou bien

b) pour préparer les dérivés 1-(cyclohexyl)-3,4-di-(hydro)-isoquinoléine de formule générale I, dans lesquels X est un reste de formule générale IV, dans lequel $R_4$ est un atome d'hydrogène, et Z représente un reste de formule générale V, $R_1$ et $R_2$ ayant la signification indiquée dans les revendications 1 à 3, on cyclise le dérivé 4-3',4'-di-(hydro)-(2'H)-isoquinoléine-1'-yl-heptane-1,7-diacide carbonique, de formule générale VII,

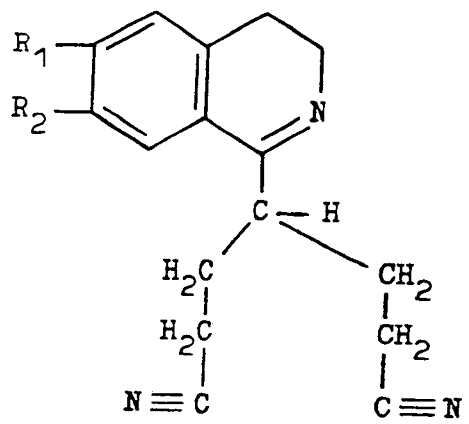

VII,

dans laquelle $R_1$ et $R_2$ ont la signification indiquée dans les revendications 1 à 3, en presence de metaux alcalins, d'alcoolates de métal alcalin, ou d'amides de métaux alcalins, ou bien:

on hydrolyse et/ou décarboxylise le dérivé 1-(cyclohexyl)-3,4-di(hydro)-isoquinoléine obtenu le cas échéant selon la variante a) de formule générale I dans lesquels Z, X, $R_1$, $R_2$ et $R_3$ ont la signification indiquée dans la variante a), en dérivés 1-(cyclohexyl)-3,4-di(hydro)-isoquinoléine correspondants de formule générale 1, dans lesquels Z est un reste de formule générale III dans lequel $R_3$ signifie l'hydrogène ou un reste de formule II, et/ou,

on transforme le cas échéant les dérivés 1-(cyclohexyl)-3,4-di(hydro)-isoquinoléine obtenus de formule générale I, dans lesquels X représente l'oxygène et Z, $R_1$, $R_2$ et $R_3$ on la signification indiquée dans les revendications 1 à 5, par réaction avec l'hydroxylamine ou l'ammoniac, en dérivés 1-(cyclohexyl)-3,4-di(hydro)-isoquinoléine correspondants dans lesquels X est un reste de formule générale IV, et out,

on transforme le cas écheant en dérivés 1-(cyclohexyl)-3,4-di(hydro)-isoquinoléine de formule générale I, le ou les substituants susceptibles de représenter $R_1$ et/ou $R_2$ en d'autres substituants susceptibles de représenter $R_1$ et/ou $R_2$, et/ou,

le cas échéant, on transforme les dérivés 1-(cyclohexyl)-3,4-di(hydro)-isoquinoléine obtenus de formule générale en sels acides d'addition, ou le cas échéant les sels acides d'addition obtenus 1-(cyclohexyl)-3,4-di(hydro)-isoquinoléine de formule générale I en dérivé libre 1-(cyclohexyl)-3,4-di(hydro)-isoquinoléine de formule générale I ou bien en d'autres sels acides d'addition.

8. Médicament, caractérisé en ce qu'il renferme 1 ou plusieurs composés selon les revendications 1 à 6 comme principe actif, et le case échéant avec 1 ou plusieurs substances adjuvantes, vectrices ou diluantes pharmaceutiques habituelles.

**Revendications pour l'Etat contractant: AT**

1. Procédé de préparation des dérivés de la 1-(cyclhexyl)-3,4-di(hydro)-isoquinoléine de formule générale I:

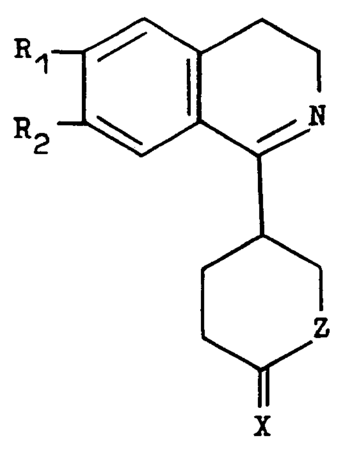

I,

dans laquelle $R_1$ et $R_2$, indépendants l'un de l'autre, représentent l'hydrogène, des groupes hydroxy, ou

alkoxy de 1 à 6 atomes de carbone, et ou bien X désigne l'oxygène et Z un reste de formule II

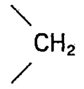

II,

ou un reste de formule III:

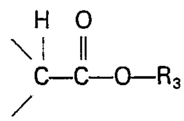

III,

dans lequel $R_3$ représente l'hydrogène, ou un reste alkyle de 1 à atomes de carbone, ou bien X signifie un reste de formule générale IV:

$$=N—R_4 \qquad IV$$

dans lequel $R_4$ est un atome d'hydrogène ou un groupe hydroxy et Z représente un reste de formule V:

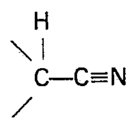

V,

un reste de formule II:

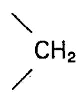

II,

ou un reste de formule générale III:

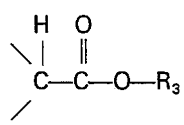

III,

dans lequel $R_3$ désigne un atome d'hydrogène ou un reste alkyle de 1 à 6 atomes de carbone, ainsi que leurs sels acides d'addition, caractérisé en ce que, de manière connue,

a) pour préparer les dérivés 1-(cyclohexyl)-3,4-di-(hydro)-isoquinoléine de formule générale I dans lesquels X représente l'oxygène et Z un reste de formule générale III dans lequel $R_3$ est un reste alkyle de 1 à 6 atomes de carbone, $R_1$ et $R_2$ ayant les significations données dans les revendications 1 à 3, on cyclise le dérivé 4-3',4'-di-(hydro)-(2'H)-isoquinoléine-1'-yl-heptane-1,7-diacide carbonique de formule générale VI:

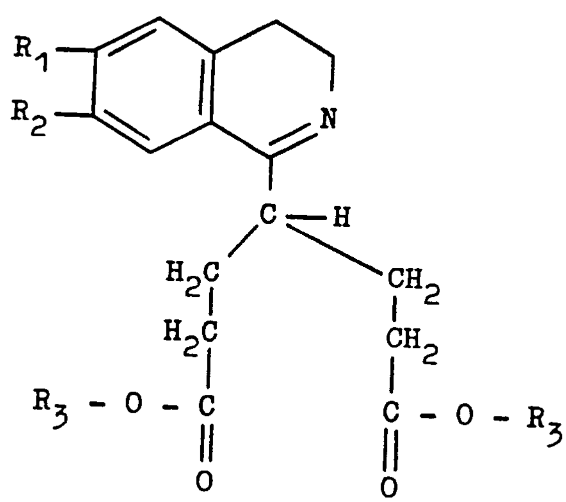

VI,

dans laquelle $R_1$ et $R_2$ ont la signification indiquée dans les revendications 1 à 5, en présence de métaux alcalins, d'alcoolates de métaux alcalins du d'amides de métaux alcalins, ou bien

b) pour préparer les dérivés 1-(cyclohexyl)-3,4-di-(hydro)-isoquinoléine de formule générale I, dans lesquels X est un reste de formule générale IV, dans lequel $R_4$ est un atome d'hydrogène, et Z représente un reste de formule générale V, $R_1$ et $R_2$ ayant la signification indiquée dans les revendications 1 à 3, on cyclise le dérivé 4-3',4'-di-(hydro)-(2'H)-isoquinoléine-1'-yl-heptane-1,7-diacide carbonique, de formule générale VII,

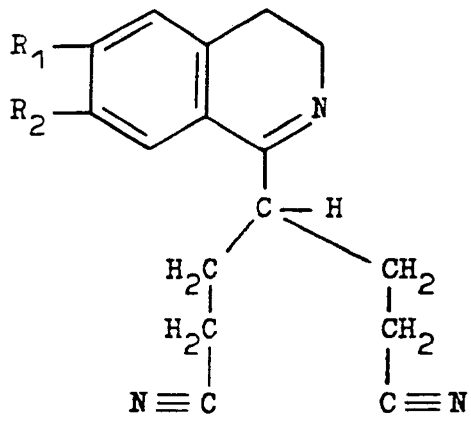

VII,

dans laquelle $R_1$ et $R_2$ ont la signification indiquée dans les revendications 1 à 3, en presence de metaux alcalins d'alcoolates de métal alcalin, ou d'amides de métaux alcalins, ou bien:

on hydrolyse et/ou décarboxylise le dérivé 1-(cyclohexyl)-3,4-di(hydro)-isoquinoléine obtenu le cas échéant selon la variante a) de formule générale I dans lesquels Z, X, $R_1$, $R_2$ et $R_3$ ont la signification indiquée dans la variante a), en dérivés 1-(cyclohexyl)-3,4-di(hydro)-isoquinoléine correspondants de formule générale 1, dans lesquels Z est un reste de formule générale III dans lequel $R_3$ signifie l'hydrogène ou un reste de formule II, et/ou,

on transforme le cas échéant les dérivés 1-(cyclohexyl)-3,4-di(hydro)-isoquinoléine obtenus de formule générale I, dans lesquels X représente l'oxygène et Z, $R_1$, $R_2$ et $R_3$ on la signification indiquée dans les revendications 1 à 5, par réaction avec l'hydroxylamine ou l'ammoniac, en dérivés 1-(cyclohexyl)-3,4-di(hydro)-isoquinoléine correspondants dans lesquels X est un reste de formule générale IV, et/ou,

on transforme le cas écheant en dérivés 1-(cyclohexyl)-3,4-di(hydro)-isoquinoléine de formule générale I, le ou les substituants susceptibles de représenter $R_1$ et/ou $R_2$ en d'autres substituants susceptibles de représenter $R_1$ et/ou $R_2$, et/ou,

le cas échéant, on transforme les dérivés 1-(cyclohexyl)-3,4-di(hydro)-isoquinoléine obtenus de formule générale en sels acides d'addition, ou le cas échéant les sels acides d'addition obtenus 1-(cyclohexyl)-3,4-di(hydro)-isoquinoléine de formule générale I en dérivé libre 1-(cyclohexyl)-3,4-di(hydro)-isoquinoléine de formule générale I ou bien en d'autres sels acides d'addition.

2. Procédé selon la revendication 1, caractérisé en ce que les dérivés de la 1-(cyclohexyl)-3,4-di(hydro)-isoquinoléine sont préparés, en ce que les restes alkoxy, représentant $R_1$ et/ou $R_2$, sont ceux comportant 1 à atomes de carbone.

3. Procédé selon la revendication 2, caractérisé en ce que les dérivés de la 1-(cyclohexyl)-3,4-di(hydro)-isoquinoléine sont préparés, en ce que les restes alkoxy représentant $R_1$ et/ou $R_2$, sont ceux comportant 1 ou 2 atomes de carbone.

4. Procédé selon la revendication 1 ou 2, caractérisé en ce que les dérivés de la 1-(cyclohexyl)-3,4-di(hydro)-isoquinoléine sont préparés, en ce que le reste alkyle, représentant $R_3$, comporte 1 à 7 atomes de carbone.

5. Procédé selon la revendication 4, caractérisé en ce que les dérivés de la 1-(cyclohexyl)-3,4-di(hydro)-isoquinoléine sont préparés, en ce que le reste alkyle, représentant $R_3$, comporte 1 ou 2 atomes de carbone.

6. Procédé selon la revendication 1, caractérisé en ce que

1-[4'(oxo)-cyclohéxyl]-3,4-di-[hydro]-6,7-di-[méthoxy)-isoquinoléine,

1-[{3'-(méthoxycarbnyl)-4-(oxo)}-cyclohex-1'-yl]-3,4-di-[hydro]-6,7-di-[méthoxy]-isoquinoléine,

1-[{3'-(éthoxycarbonyl)-4'-(oxo)}-cyclohex-1'-yl]-4,6-di-[hydro]-6,7-di-[méthoxy]-isoquinoléine,

1-[{3'-(cyano)-4'-(imino)}-cyclohex-1'-yl]-3,4-di-[hydro]-6,7-di-[méthoxy]-isoquinoléine,

1-[cyclohex-1'-yl-4'-(oxim)]-3,4-di-[hydro]-6,7-di-[méthoxy]-isoquinoléine, et

1-[{3'-(méthoxycarbonyl)-4'-(imino)}-cyclohex-1'-yl]-3,4-di-[hydro]-6,7-di-[méthoxy]-isoquinoléine,

ainsi que leurs hydrochlorures, sont préparés.

**Claims for the Contracting States: BE CH DE FR GB IT LI NL SE**

1. 1-[Cyclohexyl]-3,4-di-[hydro]-isoquinoline derivatives of the general formula

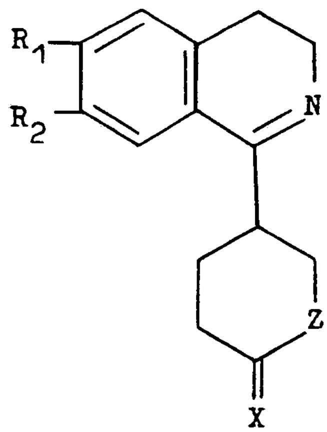

I,

wherein

$R_1$ and $R_2$, independently of one another, represent hydrogen, hydroxy groups or alkoxy radicals having from 1 to 6 carbon atoms, and either

X is oxygen and

Z is a radical of the formula

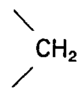

II

or a radical of the general formula

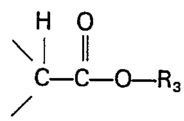

III,

wherein

$R_3$ is hydrogen or an alkyl radical having from 1 to 6 carbon atoms, or

X is a radical of the general formula

$$=N—R_4 \qquad IV,$$

wherein $R_4$ is hydrogen or a hydroxy group, and

Z is a radical of the formula

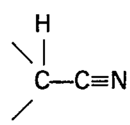

V,

a radical of the formula

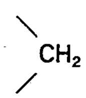

II,

or a radical of the general formula

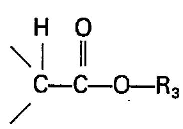

III,

wherein $R_3$ is hydrogen or an alkyl radical having from 1 to 6 carbon atoms, and acid addition salts thereof.

2. 1-[Cyclohexyl]-3,4-di-[hydro]-isoquinoline derivatives according to claim 1, characterised in that the alkoxy radicals which $R_1$ and/or $R_2$ may represent are alkoxy radicals having from 1 to 4 carbon atoms.

3. 1-[Cyclohexyl]-3,4-di-[hydro]-isoquinoline derivatives according to claim 2, characterised in that the alkoxy radicals which $R_1$ and/or $R_2$ may represent are alkoxy radicals having from 1 to 2 carbon atoms.

4. 1-[Cyclohexyl]-3,4-di-[hydro]-isoquinoline derivatives according to claim 1 or 2, characterised in that the alkyl radicals which $R_3$ may represent is an alkyl radical having from 1 to 4 carbon atoms.

5. 1-[Cyclohexyl]-3,4-di-[hydro]-isoquinoline derivatives according to claim 4, characterised in that the alkyl radical which $R_3$ may represent is an alkyl radical having 1 or 2 carbon atoms.

6. Compounds according to claim 1, characterised in that they are 1 - [4' - (oxo) - cyclohexyl] - 3,4 - di - [hydro] - 6,7 - di - [methoxy] - isoquinoline, 1 - [{3' - (methoxycarbonyl) - 4' - (oxo)} - cyclohex - 1' - yl] - 3,4 - di - [hydro] - 6,7 - di - [methoxy] - isoquinoline, 1 - [{3' - (ethoxycarbonyl) - 4' - (oxo)} - cyclohex - 1' - yl] - 3,4 - di - [hydro] - 6,7 - di - [methoxy] - isoquinoline, 1 - [{3' - (cyano) - 4' - (imido)} - cyclohex - 1' - yl] - 3,4 - di - [hydro] - 6,7 - di - [methoxy] - isoquinoline, 1 - [cyclohex - 1' - yl - 4' - (oxime)] - 3,4 - [hydro] - 6,7 - di - [methoxy] - isoquinoline and 1 - [{3' - (méthoxycarbonyl) - 4' - (imino)} - cyclohex - 1' - yl] - 3,4 - di - [hydro] - 6,7 - di - [methoxy] - isoquinoline and the hydrochlorides thereof.

7. A process for the preparation of the compounds according to claims 1 to 6, characterised in that, in a manner known *per se,*

a) for the preparation of 1-[cyclohexyl]-3,4-di-[hydro]-isoquinoline derivatives of the general formula I in which X is oxygen and Z is a radical of the general formula III wherein $R_3$ is an alkyl radical having from 1 to 6 carbon atoms and $R_1$ and $R_2$ have the same meanings in claims 1 to 3, 4-[3',4'-di-(hydro)-(2'H)-isoquinolin-1'-yl]-heptane-1,7-di-[carboxylic acid] derivatives of the general formula

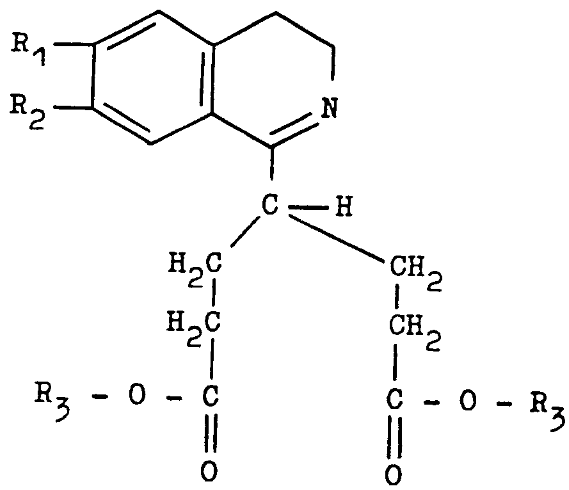

VI,

wherein $R_1$, $R_2$ and $R_3$ have the same meanings as in claims 1 to 5, are cyclised in the presence of alkali metals, alkali metal alcoholates or alkali metal amides, or

b) for the preparation of 1-[cyclohexyl]-3,4-di-[hydro]-isoquinoline derivatives of the general formula I in which X is a radical of the general formula IV wherein $R_4$ is hydrogen and Z is a radical of the formula V, and $R_1$ and $R_2$ have the same meanings as in claims 1 to 3, 4-[3',4'-di-(hydro)-(2'H)-isoquinolin-1'-yl]-heptane-1,7-di-[carboxylic and nitrile] derivatives of the general formula

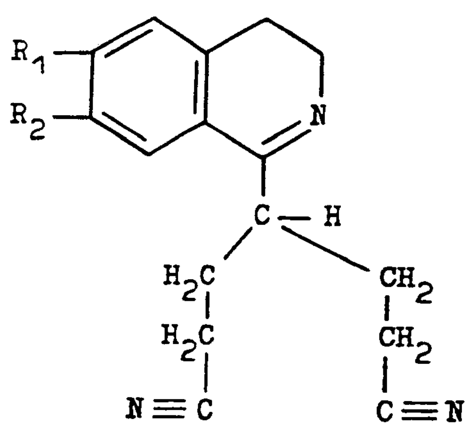

VII,

wherein $R_1$ and $R_2$ have the same meanings as in claims 1 to 3, are cyclised in the presence of alkali metals, alkali metal alcoholates or alkali metal amides, and then

optionally 1-[cyclohexyl]-3,4-di-[hydro]-isoquinoline derivatives of the general formula I obtained in accordance with Variant a) wherein Z, X, $R_1$, $R_2$ and $R_3$ have the meanings given in variant a) are hydrolysed and/or decarboxylated to form the corresponding 1-[cyclohexyl]-3,4-di-[hydro]-isoquinoline derivatives of the general formula I wherein Z is a radical of the general formula III in which $R_3$ is hydrogen or a radical of the formula II, and/or,

optionally, 1-[cyclohexyl]-3,4-di-[hydro]-isoquinoline derivatives of the general formula I which may be obtained wherein X is oxygen and Z, $R_1$, $R_2$ and $R_3$ have the same meanings as in claims 1 to 5 are converted by reaction with hydroxylamine or ammonia into the corresponding 1-[cyclohexyl]-3,4-di-[hydro]-isoquinoline derivatives of the general formula I in which X is a radical of the general formula IV, and/or

optionally, in 1-[cyclohexyl]-3,4-di-[hydro]-isoquinoline derivatives of the general formula I the substituent(s) represented by $R_1$ and/or $R_2$ is/are reacted to give (an)other substituent(s) that $R_1$ and/or $R_2$ may represent, and/or,

optionally, the resulting 1-[cyclohexyl]-3,4-di-[hydro]-isoquinoline derivatives of the general formula I are converted into acid addition salts or optionally the resulting acid addition salt of the 1-[cyclohexyl]-3,4-di-[hydro]-isoquinoline derivatives of the general formula I are converted into the free 1-[cyclohexyl]-3,4-di-[hydro]-isoquinoline derivatives of the general formula I or into other acid addition salts.

8. Medicaments, characterised by a content of one or more compounds according to claims 1 to 6 as active ingredient(s), advantageously together with one or more customary pharamaceutical carriers, extenders and/or adjusters.

**Claims for the Contracting State: AT**

1. A process for the preparation of 1-[cyclohexyl]-3,4-di-[hydro]-isoquinoline derivatives of the general formula

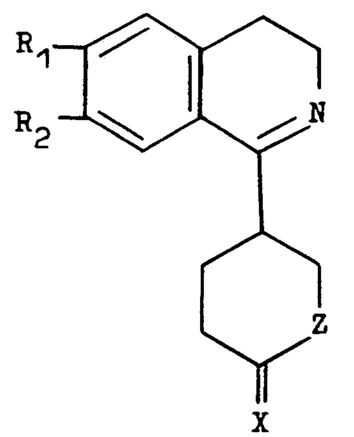

I,

wherein

$R_1$ and $R_2$, independently of one another, represent hydrogen, hydroxy groups or alkoxy radicals having from 1 to 6 carbon atoms, and either

X is oxygen and

Z is a radical of the formula

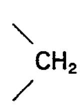

II,

or a radical of the general formula

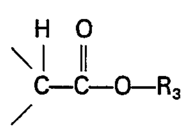

III,

wherein

$R_3$ is hydrogen or an alkyl radical having from 1 to 6 carbon atoms, or

X is a radical of the general formula

$$=N—R_4$$ IV,

wherein $R_4$ is hydrogen or a hydroxy group, and
Z is a radical of the formula

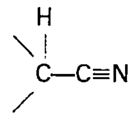

V,

a radical of the formula

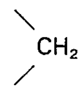

II,

or a radical of the general formula

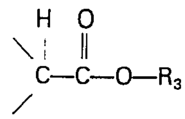

III,

wherein $R_3$ is hydrogen or an alkyl radical having from 1 to 6 carbon atoms, and acid addition salts thereof, characterised in that, in a manner known *per se*,

a) for the preparation of 1-[cyclohexyl]-3,4-di-[hydro]-isoquinoline derivatives of the general formula I in which X is oxygen and Z is a radical of the general formula III wherein $R_3$ is an alkyl radical having from 1 to 6 carbon atoms and $R_1$ and $R_2$ have the same meanings in claims 1 to 3, 4-[3',4'-di-(hydro)-(2'H)-isoquinolin-1'-yl]-heptane-1,7-di-[carboxylic acid] derivatives of the general formula

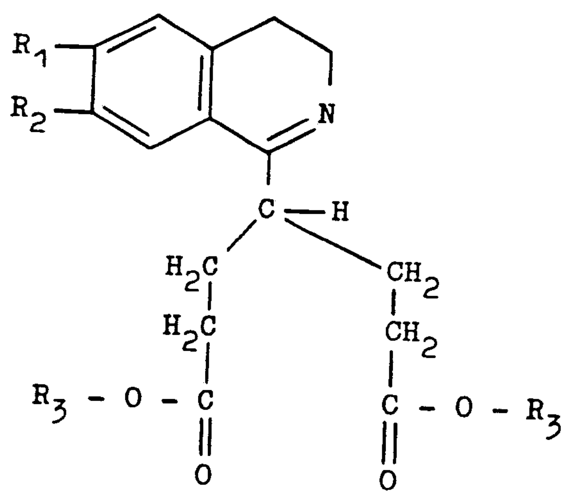

VI,

wherein $R_1$, $R_2$ and $R_3$ have the same meanings as in claims 1 to 5, are cyclised in the presence of alkali metals, alkali metal alcoholates or alkali metal amides, or

b) for the preparation of 1-[cyclohexyl]-3,4-di-[hydro]-isoquinoline derivatives of the general formula I in which X is a radical of the general formula IV wherein $R_4$ is hydrogen and Z is a radical of the formula V, and $R_1$ and $R_2$ have the same meanings as in claims 1 to 3, 4-[3',4'-di-(hydro)-(2'H)-isoquinolin-1'-yl]-heptane-1,7-di-[carboxylic and nitrile] derivatives of the general formula

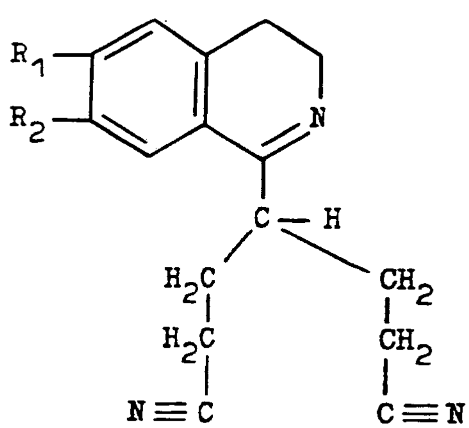

VII,

wherein $R_1$ and $R_2$ have the same meanings as in claims 1 to 3, are cyclised in the presence of alkali metals, alkali metal alcoholates or alkali metal amides, and then

optionally 1-[cyclohexyl]-3,4-di-[hydro]-isoquinoline derivatives of the general formula I obtained in accordance with Variant a) wherein Z, X, $R_1$, $R_2$ and $R_3$ have the meanings given in variant a) are hydrolysed and/or decarboxylated to form the corresponding 1-[cyclohexyl]-3,4-di-[hydro]-isoquinoline derivatives of the general formula I wherein Z is a radical of the general formula III in which $R_3$ is hydrogen or a radical of the formula II, and/or,

optionally, 1-[cyclohexyl]-3,4-di-[hydro]-isoquinoline derivatives of the general formula I which may be obtained wherein X is oxygen and Z, $R_1$, $R_2$ and $R_3$ have the same meanings as in claims 1 to 5 are converted by reaction with hydroxylamine or ammonia into the corresponding 1-[cyclohexyl]-3,4-di-[hydro]-isoquinoline derivatives of the general formula I in which X is a radical of the general formula IV, and/or,

optionally, in 1-[cyclohexyl]-3,4-di-[hydro]-isoquinoline derivatives of the general formula I the substituent(s) represented by $R_1$ and/or $R_2$ is/are reacted to give (an)other substituent(s) that $R_1$ and/or $R_2$ may represent, and/or,

optionally, the resulting 1-[cyclohexyl]-3,4-di-[hydro]-isoquinoline derivatives of the general formula I are converted into acid addition salts or optionally the resulting acid addition salts of the 1-[cyclohexyl]-3,4-di-[hydro]-isoquinoline derivatives of the general formula I are converted into the free 1-[cyclohexyl]-3,4-di-[hydro]-isoquinoline derivatives of the general formula I or into other acid addition salts.

2. A process according to claim 1, characterized in that 1-[cyclohexyl]-3,4-di-[hydro]-isoquinoline derivatives are prepared, wherein the alkoxy radicals which $R_1$ and/or $R_2$ may represent are alkoxy radicals having from 1 to 4 carbon atoms.

3. A process according to claim 2, characterized in that 1-[cyclohexyl]-3,4-di-[hydro]-isoquinoline derivatives are prepared, wherein the alkoxy radicals which $R_1$ and/or $R_2$ may represent are alkoxy radicals having from 1 to 2 carbon atoms.

4. A process according to claims 1 or 2, characterized in that 1-[cyclohexyl]-3,4-di-[hydro]-isoquinoline derivatives are prepared, wherein the alkyl radical which $R_3$ may represent is an alkyl radical having from 1 to 4 carbon atoms.

5. A process according to claim 4, characterized in that 1-[cyclohexyl]-3,4-di-[hydro]-isoquinoline derivatives are prepared, wherein the alkyl radical which $R_3$ may represent is an alkyl radical having 1 or 2 carbon atoms.

6. A process according to claim 1, characterised in that 1 - [4' - (oxo) - cyclohexyl] - 3,4 - di - [hydro] - 6,7 - di - [methoxy] - isoquinoline, 1 - [{3' - (methoxycarbonyl) - 4' - (oxo)} - cyclohex - 1' - yl] - 3,4 - di - [hydro] - 6,7 - di - [methoxy] - isoquinoline, 1 - [{3' - (ethoxycarbonyl) - 4' - (oxo)} - cyclohex - 1' - yl] - 3,4 - di - [hydro] - 6,7 - di - [methoxy] - isoquinoline, 1 - [{3' - (cyano) - 4' - (imido)} - cyclohex - 1' - yl] - 3,4 - di - [hydro] - 6,7 - di - [methoxy] - isoquinoline, 1 - [cyclohex - 1' - yl - 4' - (oxime)] - 3,4 - [hydro] - 6,7 - di - [methoxy] - isoquinoline and 1 - [{3' - (méthoxycarbonyl) - 4' - (imino)} - cyclohex - 1' - yl] - 3,4 - di - [hydro] - 6,7 - di - [methoxy] - isoquinoline and the hydrochlorides thereof are prepared.